# EUROPEAN PATENT SPECIFICATION

(11) **EP 4 224 164 B1**
(45) Date of publication and mention of the grant of the patent: **10.09.2025**
(21) Application number: 21874763.2
(22) Date of filing: 10.02.2021
(51) Int. Cl.: G01N 35/00, G16H 20/10, G16H 50/20, G16H 50/30, G16H 20/40

(54) **DATA PROCESSING DEVICE AND AUTOMATED ANALYZER**
DATENVERARBEITUNGSVORRICHTUNG UND AUTOMATISIERTER ANALYSATOR
DISPOSITIF DE TRAITEMENT DE DONNÉES ET ANALYSEUR AUTOMATISÉ

(30) Priority: 30.09.2020 JP 2020164663
(43) Date of publication of application: 09.08.2023
(73) Proprietor: HITACHI HIGH-TECH CORPORATION, Tokyo 105-6409 (JP)
(72) Inventor: SAGAE, Nozomi, Tokyo 105-6409 (JP); IIJIMA, Masahiko, Tokyo 105-6409 (JP)
(74) Representative: Strehl & Partner mbB
(86) International application number: PCT/JP2021/004905
(87) International publication number: WO 2022/070455

(56) References cited:
- WO-A1-2010/067513
- JP-A- 2000 187 037
- JP-A- 2008 032 493
- JP-A- 2011 007 686
- JP-A- 2011 047 911
- JP-A- 2018 124 171
- US-A1- 2005 261 840
- US-A1- 2015 219 556
- US-B2- 9 506 942

## Description

### Technical Field

The present invention relates to data processing device according to claim 1.

### Background Art

For example, an automatic analyzer having a function of executing biochemical analysis, immunological analysis, or the like for a clinical test processes data obtained by measurement of a biological sample (also referred to as "specimen") such as blood or urine. The automatic analyzer measures a time change in absorbance, for example, in a reaction liquid where a sample and a reagent are mixed at a given ratio and stirred to cause a reaction. As a result, the automatic analyzer calculates, for example, a concentration or an activity value of a target material in the sample as a measured value. In addition, the automatic analyzer measures, for example, a standard solution for correcting a reagent or a quality control sample for checking a state of a reagent per device or analysis item to grasp the state of the device, the sample and to suppress a decrease in measurement accuracy.

Prior art examples of the automatic analyzer described above include JP2009-204448A (PTL 1). PTL 1 discloses an automatic analyzer that acquires a reaction rate constant using an approximate expression from reaction process data representing an absorbance change that is stored in a time series, and determines whether or not the reaction is abnormal based on the value of the reaction rate constant.

### Citation List

### Patent Literature

PTL 1: JP2009-204448A

**Further,** US 9 506 942 B2 **discloses an automatic analyzer from which the precharacterizing part of claim 1 starts out. Related art is disclosed in** US 2015/219556 A1**.**

### Summary of Invention

### Technical Problem

Performance of the automatic analyzer such as measurement accuracy is considered to be determined depending on a combination of a plurality of performance controlling factors, in other words, a plurality of elements that affect the performance. Examples of the performance controlling factors include a condition or a state of installation of the automatic analyzer, a state of a sample or a reagent, and a state of an operation of an operator. Examples of the performance controlling factors include the amount or accuracy of a sample or a reagent dispensed into a reaction container and the homogeneity or stability of a sample or a standard solution.

Accordingly, when the measurement result is abnormal, the automatic analyzer and the operator need to improve the abnormal state and to suppress a decrease in measurement accuracy by identifying a cause for the abnormality and taking a countermeasure such as maintenance to improve the cause. The cause and the performance controlling factor have a certain relationship.

The automatic analyzer in the prior art example can determine and detect whether or not the measurement result is abnormal, for example, using a method of comparing the value of the reaction process data to a threshold representing a predetermined range in a normal state. However, it is difficult to identify the cause for the abnormality because a plurality of performance controlling factors can be considered as the cause. There may be a case where it is difficult for the operator to identify the cause of the abnormality or a case where a large amount of time and effort is required for a specific operation. PTL 1 does not describe the identification of the cause for the abnormality. In particular, in an automatic analyzer for a clinical test in the related art, it is extremely difficult for an operator to rapidly or easily identify whether or not abnormality occurs in measurement of a plurality of analysis target samples having unknown concentrations and a cause for the abnormality and to improve the abnormality. Regarding this point, there is room for improvement.

An object of the present invention is to provide a data processing device according to claim 1 configured to rapidly or easily identify a cause for abnormality of measurement and to improve the abnormality. Solution to Problem

**The invention solving the above problem is defined by the appended claims.**

A representative embodiment of the present invention has the following configurations. A data processing device according to an embodiment is configured to execute the following processes of: referring to data regarding measurement of a sample by an automatic analyzer; calculating a combination of values of a plurality of factors based on reaction process data among the data, the factors being predetermined variables or measured values representing characteristics of a reaction; referring to a correspondence table including a distribution diagram and information regarding an abnormality cause candidate, the distribution diagram including a normal range region and a plurality of abnormal range regions that are generated by a combination of normal/abnormal ranges of the plurality of factors, and the abnormality cause candidate being associated with each of the abnormal range regions; determining an abnormal range region in the correspondence table to which the combination of the values of the plurality of factors corresponds; and outputting the information regarding the abnormality cause candidate associated with the corresponding abnormal range region to a user.

### Advantageous Effects of Invention

According to the representative embodiment of the present invention, it is possible to provide a data processing device configured to rapidly or easily identify a cause for abnormality of measurement and to improve the abnormality.

### Brief Description of Drawings

[Fig. 1] Fig. 1 illustrates a configuration of a system including a data processing device and an automatic analyzer according to a first embodiment of the present disclosure.
[Fig. 2] Fig. 2 illustrates a configuration example of the automatic analyzer according to the first embodiment.
[Fig. 3] Fig. 3 illustrates a configuration example of the data processing device according to the first embodiment.
[Fig. 4] Fig. 4 illustrates the flow of a main process of the data processing device according to the first embodiment.
[Fig. 5] Fig. 5 illustrates a concept and a first configuration example of a correspondence table in the first embodiment"
[Fig. 6] Fig. 6 illustrates a concept and a second configuration example of the correspondence table in the first embodiment.
[Fig. 7] Fig. 7 illustrates a concept and a third configuration example of the correspondence table in the first embodiment.
[Fig. 8] Fig. 8 illustrates an example of reaction process data in the first embodiment.
[Fig. 9] Fig. 9 illustrates an example of a distribution diagram of a combination of two factors based on the reaction process data of Fig. 8 in the first embodiment.
[Fig. 10] Fig. 10 illustrates an example of the reaction process data including data that is distributed in an abnormal range region #1 of Fig. 9 in the first embodiment.
[Fig. 11] Fig. 11 illustrates an example of a screen that outputs cause candidates and the like associated with the abnormal range region #1 of Fig. 9 in the first embodiment.
[Fig. 12] Fig. 12 illustrates a screen example in a first modification example of the first embodiment.
[Fig. 13] Fig. 13 illustrates an example of the reaction process data in a second modification example of the first embodiment.
[Fig. 14] Fig. 14 illustrates an example of a distribution diagram of a combination of two factors based on the reaction process data of Fig. 13 in the second modification example.
[Fig. 15] Fig. 15 illustrates an example of the reaction process data including data that is distributed in an abnormal range region #1 of Fig. 14 in the second modification example.
[Fig. 16] Fig. 16 illustrates an example of the reaction process data in a third modification example of the first embodiment.
[Fig. 17] Fig. 17 illustrates an example of a distribution diagram of a combination of two factors based on the reaction process data of Fig. 16 in the third modification example.
[Fig. 18] Fig. 18 illustrates a screen example that outputs cause candidates and the like associated with the abnormal range region #1 of Fig. 17 in the third modification example.

### Description of Embodiments

Hereinafter, an embodiment of the present invention will be described in detail based on the drawings. In all of the drawings, in principle, the same components are represented by the same reference numerals, and the repeated description thereof will not be made. For the components (including steps and the like) in the embodiment other than essential components, addition, deletion, replacement, or the like can be made.

### <First Embodiment>

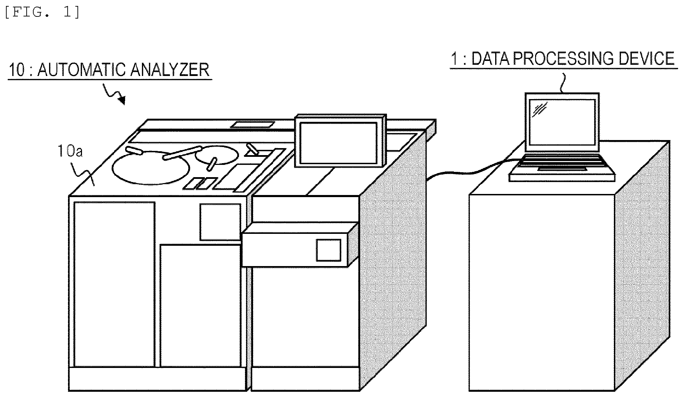

An automatic analyzer and a data processing device according to a first embodiment of the present disclosure will be described using Figs. 1 to 11.

The data processing device according to the first embodiment calculates a plurality of factors based on reaction process data that is obtained in measurement of a sample in the automatic analyzer, the factors being predetermined variables representing characteristics of a reaction. The reaction process data is data representing, in a time series, a state of a reaction or the like of a sample and a reagent that is obtained in measurement of absorbance. The reaction process data is, for example, a graph where the horizontal axis represents the time and the vertical axis represents the absorbance. For convenience of description, the factor is a factor relating to identification of a cause for abnormality. A specific correspondence table is configured by a combination of a plurality of factors. This factor is a concept different from the above-described performance controlling factor.

The data processing device determines or checks whether a factor value of each of the factors is normal or abnormal. In other words, the data processing device classifies the factor values into a normal range or an abnormal range. Using the determination results of the factor values, the data processing device generates a combination (referred to as "first combination") of normal/abnormal ranges of the plurality of factors as a distribution diagram. This distribution diagram is configured as a two- or higher dimensional region including two or more factors. The distribution diagram includes a normal range region and plurality of abnormal range regions. The data processing device generates the correspondence table including the distribution diagram.

The data processing device associates information regarding a cause candidate with each of the abnormal range regions in the first combination of the factors in the correspondence table. In other words, in the correspondence table, a combination (referred to as "second combination") of the cause candidates is associated with the first combination of the factors. The cause candidates are candidates of causes that are estimated as a cause for abnormality. The cause candidates are various elements relating to the above-described performance controlling factors. Examples of the cause candidates include a condition or a state of installation of the automatic analyzer, a state of a sample or a reagent, and a state of an operation of an operator.

In addition, the data processing device may associate information regarding a countermeasure with each of the cause candidates in the correspondence table. The information regarding the countermeasure is information regarding maintenance or the like for improving or resolving a state of abnormality. The information regarding the countermeasure is information regarding a guidance, a troubleshooting, an alert, a manual, or the like to the operator that represents a countermeasure against a cause of abnormality.

In the data processing device, the correspondence table described above may be set in advance. When abnormality relating to measurement is detected or checked based on measurement data from the automatic analyzer, the data processing device determines the abnormal range region to which the factor values corresponding to the abnormality correspond based on the correspondence table. The data processing device outputs information regarding a cause candidate or a countermeasure associated with the abnormal range region to the operator. As a result, using the output information as a support, the operator can identify a cause for the abnormality or take a countermeasure against the abnormality more rapidly and easily than in the related art.

### [Automatic Analyzer and Data Processing Device]

Fig. 1 illustrates a configuration of a system (in other words, an automatic analysis system) including the data processing device and the automatic analyzer according to the first embodiment. The system of Fig. 1 is mainly configured by a data processing device 1 and an automatic analyzer 10 according to the first embodiment, and these components are connected through wired or wireless connection. The external appearance of the automatic analyzer 10 or the data processing device 1 illustrated in the drawing is merely exemplary. The operator is a user such as an engineer and operates the automatic analyzer 10 or the data processing device 1 to execute an operation.

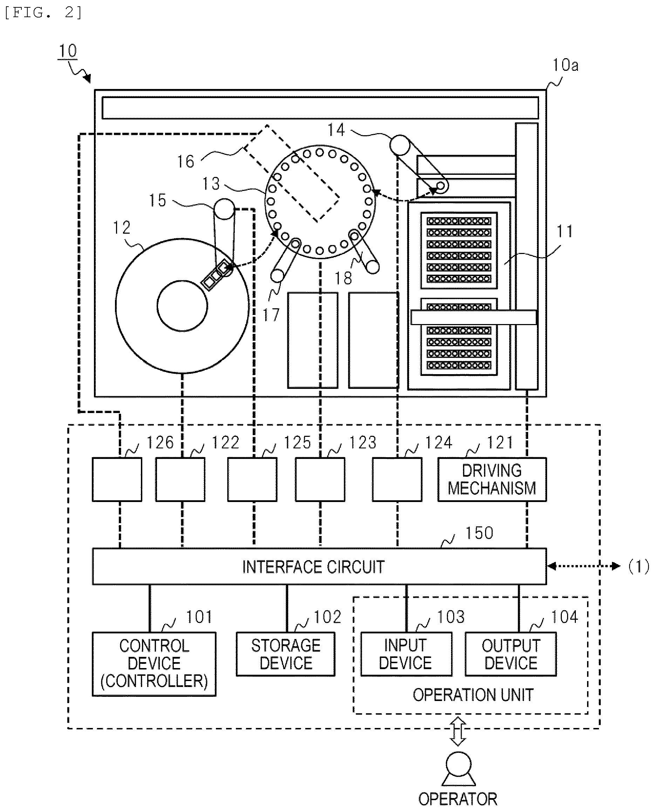

The automatic analyzer 10 is an automatic analyzer for a clinical test and has a function of executing a process such as measurement or analysis on a biological sample, for example, as a biochemical analysis function. The automatic analyzer 10 has, for example, a function of measuring the concentration or the like of a component material of a sample. The automatic analyzer 10 is configured by, for example, a single module or a combination of a plurality of modules. When the automatic analyzer 10 is configured by a combination of modules, for example, the automatic analyzer 10 can be configured by a combination of one or more analysis modules, an operation module, and a transport module. In an upper surface 10a of a casing of the automatic analyzer 10, various mechanisms (Fig. 2) described below are disposed.

The data processing device 1 is a device having a function of receiving and acquiring data regarding measurement, and the like of a sample from the automatic analyzer 10 through communication and processing or analyzing the data. The data includes measurement data such as a measurement result. In particular, the data processing device 1 has a function of providing information regarding a cause, a countermeasure, and the like for abnormality of the measurement to the operator as a support based on the analysis or the check of the measurement result. The data processing device 1 can be implemented as, for example, a computer such as a PC. Alternatively, the data processing device 1 may be implemented as a part of the automatic analyzer 10, for example, a part of an operation module or a control device. The data processing device 1 may be disposed in the vicinity of the automatic analyzer 10 as illustrated in the drawing, or may be disposed distant from the automatic analyzer 10 through a LAN or the like.

In the first embodiment, the automatic analyzer 10 and the data processing device 1 are separately configured, and a specific function for abnormality cause identification support is mounted on the data processing device 1. However, the present invention is not limited to this configuration. An integrated apparatus having both of the function of the automatic analyzer 10 and the function of the data processing device 1 may be implemented.

### [Automatic Analyzer]

Fig. 2 illustrates a configuration example of the automatic analyzer 10. The upper portion of Fig. 2 illustrates main elements disposed in the upper surface 10a of the casing of the automatic analyzer 10, and the lower portion of Fig. 2 schematically illustrates elements in the casing that are connected to the elements illustrated in the upper portion. The automatic analyzer that is applicable is not limited to this configuration example.

The automatic analyzer 10 includes a sample holding mechanism 11, a reagent holding mechanism 12, a reaction container holding mechanism 13, a sample dispensing mechanism 14, a reagent dispensing mechanism 15, an optical measurement mechanism 16, a stirring mechanism 17, and a cleaning mechanism 18. In addition, the automatic analyzer 10 includes a sample transport mechanism or a disposing mechanism.

The sample holding mechanism 11 has, for example, a box shape such as a rack and is a mechanism that can hold and transport a plurality of sample containers. The sample holding mechanism 11 may be a disk-shaped mechanism or may be prepared as another module that can be additionally connected. The sample container contains a specimen such as blood as a sample. The sample container may be placed on a sample rack which mounts one sample container or a plurality of sample containers and may be transported. The sample holding mechanism 11 also includes, for example, a transport mechanism that can transport the sample containers and the like by a parallel movement or a rotational movement. A driving mechanism 121 is connected to the sample holding mechanism 11. The sample holding mechanism 11 in Fig. 2 has functions of transporting the introduced sample rack to the sample dispensing mechanism 14, holding the sample rack for which dispensing is completed until analysis completion, and collecting the sample rack for which analysis is completed to a predetermined position.

The reagent holding mechanism 12 is, for example, a disk-shaped mechanism and is a mechanism that can hold and transport a plurality of reagent containers. The reagent container contains a reagent solution. The reagent container may be configured as a reagent bottle or the like that collectively contains a plurality of reagent containers. The reagent holding mechanism 12 may be a rack-shaped mechanism or may be prepared as another module. A driving mechanism 122 is connected to the reagent holding mechanism 12. The sample holding mechanism 11 and the reagent holding mechanism 12 may be configured as an integrated common mechanism.

The reaction container holding mechanism 13 is, for example, a disk-shaped mechanism and is a mechanism that can hold and transport a plurality of reaction containers. The reaction container holding mechanism 13 includes a thermostat. In the reaction container holding mechanism 13, a plurality of reaction containers can be placed on, for example, a circumference of a disk. A driving mechanism 123 is connected to the reaction container holding mechanism 13. The reaction container is formed of a translucent material. A sample and a reagent are dispensed (aspirated and discharged) into the reaction container from the sample container and the reagent container, and a mixed liquid thereof is stirred or cultured to obtain a reaction liquid as a measurement target. The reaction container is maintained at a predetermined temperature by the thermostat.

The disk-shaped mechanism such as the reaction container holding mechanism 13 includes a rotation mechanism, and can move the containers provided at positions on the circumference by repeating rotation and stop in a circumferential direction per predetermined distance. As a result, the containers on the disk are sequentially moved to predetermined positions, and a predetermined process is executed thereon by a mechanism corresponding to each of the positions.

The sample dispensing mechanism 14 is disposed in the vicinity of the sample holding mechanism 11 and the reaction container holding mechanism 13, and is a mechanism that dispenses (aspirates and discharges) the sample in the sample container at a dispensing position of the sample holding mechanism 11 into a predetermined reaction container of the reaction container holding mechanism 13. The sample dispensing mechanism 14 includes, for example, an arm or a probe. A driving mechanism 124 is connected to the sample dispensing mechanism 14.

The reagent dispensing mechanism 15 is disposed in the vicinity of the reagent holding mechanism 12 and the reaction container holding mechanism 13, and is a mechanism that dispenses (aspirates and discharges) the reagent in the reagent container of the reagent holding mechanism 12 into the reaction container of the reaction container holding mechanism 13. The reagent dispensing mechanism 15 includes, for example, an arm or a pipette nozzle. A driving mechanism 125 is connected to the reagent dispensing mechanism 15.

The stirring mechanism 17 stirs the mixed liquid of the sample and the reagent in the reaction container of the reaction container holding mechanism 13 to accelerate the reaction. The reaction container containing the reaction liquid may be moved to a predetermined region and cultured. The stirring mechanism 17 may be integrated with the dispensing mechanism.

The optical measurement mechanism 16 includes a light source, a photometer, and a measurement circuit. The optical measurement mechanism 16 is disposed at a predetermined position of the reaction container holding mechanism 13. The optical measurement mechanism 16 irradiates the reaction container with light from the light source and detects light transmitted through or scattered from the reaction container with the photometer. The photometer is, for example, a spectrophotometer that can measure an absorbance based on spectroscopic detection. A signal (for example, an analog signal of scattered light) for each of the samples that is detected by the photometer undergoes a process such as analog-digital conversion in a measurement circuit and is transmitted to a control device 101 as a measurement signal. A driving mechanism 126 is connected to the optical measurement mechanism 16. The optical measurement mechanism 16 may be provided at another position irrespective of the position of the reaction container holding mechanism 13.

The cleaning mechanism 18 is used for cleaning a reusable type reaction container or the like. In a clinical test, a disposable type reaction container, dispensing tip, or the like may be used. The used disposable type reaction container or the like is disposed to a predetermined position by a disposing mechanism. In this case, the automatic analyzer 10 includes a mechanism that contains the disposable type reaction container or dispensing tip.

In the casing of the automatic analyzer 10, a mechanism such as a pump or a tank, a power supply mechanism, and the like are further provided. The pump configures the dispensing mechanism or the like. Examples of the tank include each of tanks that stores system water, a cleaning solution, a waste solution, and the like.

Each of the units such as the sample holding mechanism 11 is connected to the corresponding driving mechanism as schematically indicated by a broken line in the drawing. For example, the driving mechanism 121 is a mechanism including a motor, a circuit, or the like for driving a predetermined operation of the sample holding mechanism 11. Each of the driving mechanisms is connected to an interface circuit 150. The interface circuit 150 is a portion that enables electrical connection and communication between the respective units. The interface circuit 150 includes a communication interface device having a predetermined communication interface and is also connected to the data processing device 1 of Fig. 1.

The control device 101 as a controller, a storage device 102, an input device 103, an output device 104, a power supply device (not illustrated), and the like are connected to the interface circuit 150. The power supply device supplies power to each of the units. Predetermined units can communicate with each other through the interface circuit 150. The input device 103 and the output device 104 configure an operation unit for allowing the operator to operate the automatic analyzer 10. The input device 103 may be an operation panel or the like including buttons. The output device 104 may be a display device or a printing device. The operation unit may be configured as a touch panel or the like by integrating the input device 103 and the output device 104 with each other. The operation unit may be configured as an operation module. Alternatively, the data processing device 1 of Fig. 1 may function as the operation unit. In addition, the operation unit may include a light emitting device such as a LED or a voice output device such as a speaker. As a result, a notification or an alert can be output to the operator.

The operation of the operator includes an operation of identifying a cause for abnormality or taking a countermeasure against the abnormality. The operator operates the operation unit of the automatic analyzer 10 to execute the operation during analysis and, for example, when abnormality of measurement occurs, operates the data processing device 1 to execute the operation of identifying a cause for abnormality or taking a countermeasure against the abnormality.

The control device 101 is configured as an IC substrate, a computer, or the like. The control device 101 controls the entire automatic analyzer 10 to implement an automatic analysis function. For example, during measurement and analysis of a specimen, the control device 101 controls the operation of each of the units such as the sample holding mechanism 11 by transmitting a control signal to each of the driving mechanisms based on an operation of the operator, setting information, analysis request information, and the like. The control device 101 includes, for example, a CPU, a ROM, and a RAM and implements a control function through a process of a circuit or a program.

The storage device 102 is configured as an internal or external non-volatile storage device or the like and is, for example, a semiconductor memory or a hard disk. The storage device 102 stores various data such as a program, setting information, analysis request information, measurement data, and analysis result information.

The operator registers a test item of each specimen of a patient or the like and registers an analysis request in an analysis item corresponding to the test item through a screen of the operation unit of the automatic analyzer 10 or a screen of the data processing device 1. Registration information is stored in the storage device 102. The operator inputs an instruction of analysis of the specimen. The control device 101 starts the measurement and analysis of the specimen while referring to the registration information in accordance with the instruction.

The control device 101 controls the optical measurement mechanism 16 or the like to measure an absorbance for each specimen (corresponding to the reaction container), acquires the measurement signal, and stores the measurement signal in the storage device 102. The measurement signal includes reaction process data that is time series data. The control device 101 analyzes the designated analysis item using the data of the measurement signal to calculate a measured value such as a concentration of a component of the specimen. During the measurement and analysis, the control device 101 calculates, for example, the value of the concentration of the component based on a preset analysis method and a calibration curve of the analysis item.

The control device 101 causes the storage device 102 to store data including the result of the measurement and analysis of each specimen, that is, the reaction process data including the absorbance or data including the measured value such as the concentration. The control device 101 can display the result on, for example, a display screen of the output device 104. The operator can check the measurement and analysis result information through the screen. In addition, the control device 101 may determine, for example, whether or not abnormality occurs in the measurement result. For example, the control device 101 compares the measured value and a condition relating to the abnormality determination to each other. Alternatively, the control device 101 compares a value of a predetermined variable representing a characteristic of the reaction in the reaction process data and the condition relating to the abnormality determination to each other. The condition is, for example, a threshold that configures a normal range. The control device 101 determines, for example, whether the variable value is in a normal range or in an abnormal range. In this case, the data regarding the measurement and analysis result by the automatic analyzer 10 includes determination result information representing whether or not abnormality occurs.

The data processing device 1 acquires the data regarding the measurement and analysis result described above from the control device 101 of the automatic analyzer 10 and executes an abnormality cause identification support process as a specific process. Alternatively, instead of the control device 101, the data processing device 1 may determine whether the measured value or the variable value is normal or abnormal based on the data regarding the measurement and analysis result.

### [Data Processing Device]

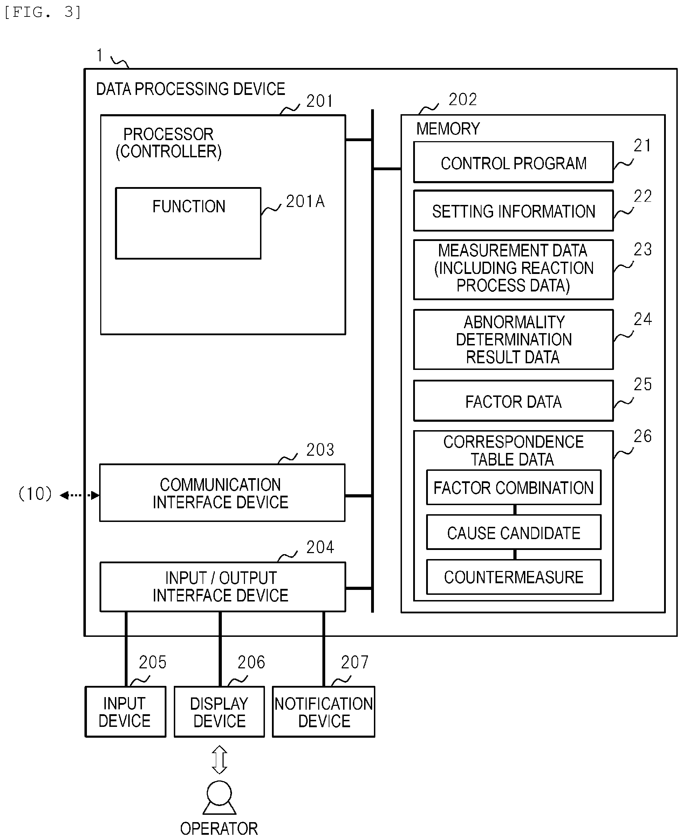

Fig. 3 illustrates a configuration example as a computer system including the data processing device 1 according to the first embodiment. The computer system includes the data processing device 1 and an input device 205, a display device 206, and a notification device 207 that are connected to the data processing device 1. The data processing device 1 is configured by a processor 201, a memory 202, a communication interface device 203, an input/output interface device 204, a bus that connects the above components to each other, and the like. The input device 205, the display device 206, the notification device 207, and the like are connected to the input/output interface device 204. The input device 205 is, for example, a keyboard or a mouse. The display device 206 is, for example, a liquid crystal display. The notification device 207 is, for example, a light emitting device or a voice output device that can give notification to the operator. However, the present invention is not limited to this configuration, and the input device 205, the display device 206, or the notification device 207 may also be configured to be integrated with main body of the data processing device 10. The communication interface device 203 is connected to the interface circuit 150 of the automatic analyzer 10 of Fig. 2, and communicates with the control device 101 and the like of the automatic analyzer 10 through a predetermined communication interface.

The processor 201 is configured by, for example, a CPU, a ROM and a RAM, and configures the controller of the data processing device 1. The processor 201 implements a predetermined function 201A based on a software program process. The function 201A is a function of supporting the operation of the operator when abnormality of measurement occurs in the automatic analyzer 10, and includes a function of outputting information regarding cause candidates or countermeasures for abnormality. The processor 201 receives and acquires, for example, the data regarding the measurement and analysis result including measurement data 23 from the automatic analyzer 10 and stores the data in the memory 202. The processor 201 refers to information regarding a condition including an analysis parameter of the analysis item of the sample based on the measurement data 23 and stores the information in the memory 202. The processor 201 executes abnormality determination regarding whether or not the measured value or the variable value is abnormal or check the abnormality determination result based on the measurement data 23. The processor 201 calculates or extracts a value of a predetermined factor based on the reaction process data in the measurement data 23. The processor 201 calculates or extracts values of a plurality of factors that are predetermined variables representing characteristics of the reaction in the reaction process data.

The processor 201 stores, as correspondence table data 26, a correspondence table that is set in advance by default. In the correspondence table, the first combination of normal/abnormal ranges of the plurality of factor values and the second combination of information regarding a cause candidate and a countermeasure associated with each of the range regions of the first combination are set. Based on the measurement data 23 and, when abnormality of measurement is detected or checked, based on the correspondence table data 26, the processor 201 determines an abnormal range region to which factor values corresponding to the abnormality correspond. The processor 201 displays a screen including the information regarding the cause candidates and the countermeasures associated with the abnormal range region to the operator.

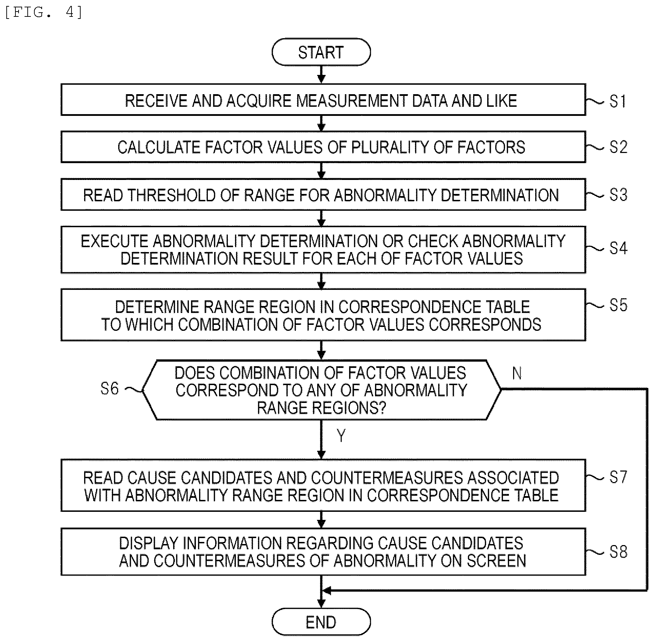

The memory 202 is configured by a non-volatile storage device or the like and stores various data or information used in the processor 201 or the like. The memory 202 is, for example, a semiconductor memory or a hard disk. The memory 202 stores a control program 21, setting information 22, the measurement data 23, abnormality determination result data 24, factor data 25, the correspondence table data 26, and the like. The control program 21 is a program for implementing the function 201A and the like of the data processing device 1. The processor 201 executes the process (Fig. 4 described below) corresponding to the function 201A based on the control program 21. The setting information 22 is setting information of the control program 21 or a user. The operator or a manufacturer person can operate the input device 205 to set necessary information while seeing a screen of the display device 206, and the information is reflected on the setting information 22 and the like.

The measurement data 23 is measurement data including the reaction process data during the measurement that can be acquired by communication from the automatic analyzer 1. The measurement data 23 also includes information regarding a condition such as an analysis parameter relating to measurement and analysis. The reaction process data of the measurement data 23 or the abnormality determination result data 24 includes a value of a factor such as a predetermined variable or measured value or information from which the factor can be calculated. The abnormality determination result data 24 is determination result information representing whether the result is normal or abnormal during measurement by the control device 101 or the processor 201. The factor data 25 is data regarding a factor value that is generated and stored based on the reaction process data of the measurement data 23 or the abnormality determination result data 24.

The abnormality determination result or the factor value may be calculated by the control device 101 and described in the measurement data 23 or the like. However, the present invention is not limited to this configuration. The data processing device 1 may execute the abnormality determination or calculate the factor value based on the measurement data 23 or the like.

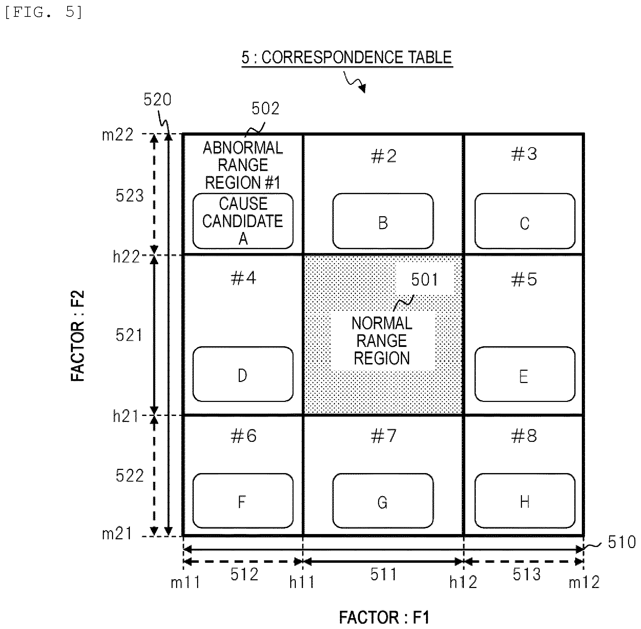

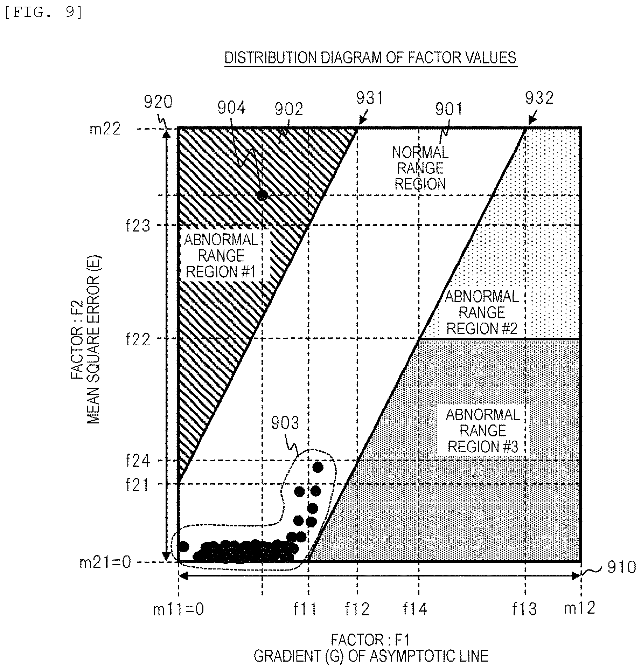

The correspondence table data 26 is data regarding the correspondence table where the first combination of normal/abnormal ranges of the plurality of predetermined factor and the second combination of information regarding cause candidates or countermeasures are associated with each other. The correspondence table data 26 includes the distribution diagram that is generated by the combination of the normal/abnormal ranges of the factor values described below (Fig. 5 or Fig. 9). This distribution diagram may be an image, a graph, a matrix, or the like, and the form thereof is not limited.

The computer system is not limited to the configuration example illustrated in Fig. 3 and can adopt various configurations. For example, a storage device, a server device, or the like may be connected to the data processing device 1, and various data may be stored in the storage device, the server device, or the like. In the data processing device 1, the control program 21 may be distributed from the server device or the like and installed. A client terminal device (for example, a PC) that is operated by the operator may be connected to the data processing device 1 through a communication network for client-server communication. In a cloud computing system or the like on the communication network, the functions of the data processing device 1 may be implemented. The computer program may be stored in a non-transitory computer-readable recording medium.

The data processing device 1 is connected by communication to the automatic analyzer 10, for example, at all times and executes data processing in real time in cooperation with the control device 101. As a result, when abnormality of measurement occurs, the data processing device 1 supports the operator instantaneously. The present invention is not limited to this configuration. The data processing device 1 may be connected by communication to the automatic analyzer 10 only for a predetermined period of time, for example, when an instruction is input from the operator. The data processing device 1 may be connected by communication to the automatic analyzer 10 by transmitting a request or an instruction from the data processing device 1 to the automatic analyzer 10 or by transmitting a request or an instruction from the automatic analyzer 10 to the data processing device 1.

The detailed functional blocks that are implemented by the processor 201 of the data processing device 1 of Fig. 3 are not illustrated but, for example, are as follows. The data processing device 1 includes a data reception unit, a data processing unit, a data input unit, a factor determination unit, and a data display unit. The data reception unit receives and acquires data regarding the measurement and analysis result from the automatic analyzer 10 during or after measurement, and stores the data in the memory 202 as the measurement data 23 or the abnormality determination result data 24. The data input unit receives an input of setting information or an instruction from a person such as the operator that is the user, and stores the input information in the memory 202. The data display unit displays or notifies information regarding a cause candidate or a countermeasure associated with the abnormality to the operator based on the correspondence table.

The data processing unit executes predetermined data processing on the acquired data, in particular, a process relating to the abnormality cause identification support using the correspondence table. The data processing unit determines or checks whether or not abnormality occurs based on the measurement data 23 or the like. The data processing unit calculates a combination of factor values that are predetermined variables based on the reaction process data. When abnormality occurs, the data processing unit determines the abnormal range region to which the combination of the factor values corresponding to the abnormality corresponds by comparing the factor values to the correspondence table. The data processing unit determines a cause candidate or a countermeasure associated with the abnormal range region from the correspondence table and generates output information.

### [Process Flow]

Fig. 4 illustrates the flow of a main process of the processor 201 of the data processing device 1 of Fig. 3. The flow of Fig. 4 includes Steps S1 to S8. The flow includes processes of calculating a combination of values of two factors, in particular, based on the measurement data 23 or the like from the automatic analyzer 10 and comparing the factor values to the correspondence table (correspondence table data 26) to output the cause candidates or the like. In the example, as the factor, a predetermined variable based on the reaction process data is used.

In Step S1, the data processing device 1 receives and acquires the data regarding the measurement and analysis result from the automatic analyzer 10 and stores the data in the memory 202 as the measurement data 23 or the like.

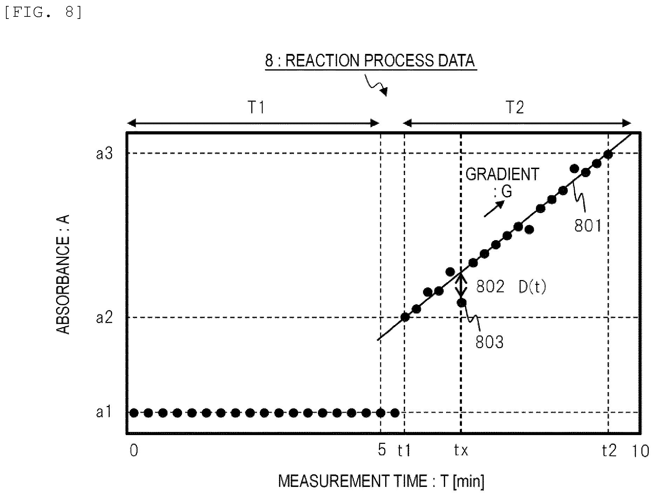

In Step S2, the data processing device 1 calculates values of a plurality of factors that are a plurality of predetermined variables based on the reaction process data (Fig. 8 described below) in the measurement data 23, and stores the factor values in the memory 202 as the factor data 25 (Fig. 3). In the example, the data processing device 1 calculates values of two factors (represented by F1 and F2). When the measurement data 23 includes the factor values, the calculation can be skipped.

In Step S3, the data processing device 1 reads, from the memory 202, a threshold of normal/abnormal ranges of each of the factors that configure a condition of abnormality determination based on the abnormality determination result data 24. Examples of the threshold include an upper limit threshold and a lower limit threshold that configure the normal range, but the threshold is not limited thereto. The abnormality determination result data 24 obtained from the automatic analyzer 10 includes information regarding the threshold of the range in the condition. When data from the automatic analyzer 10 does not include the information regarding the threshold, the data processing device 1 may refer to the setting information of the automatic analyzer 10 or the setting information of the data processing device 1 to acquire information regarding the same condition.

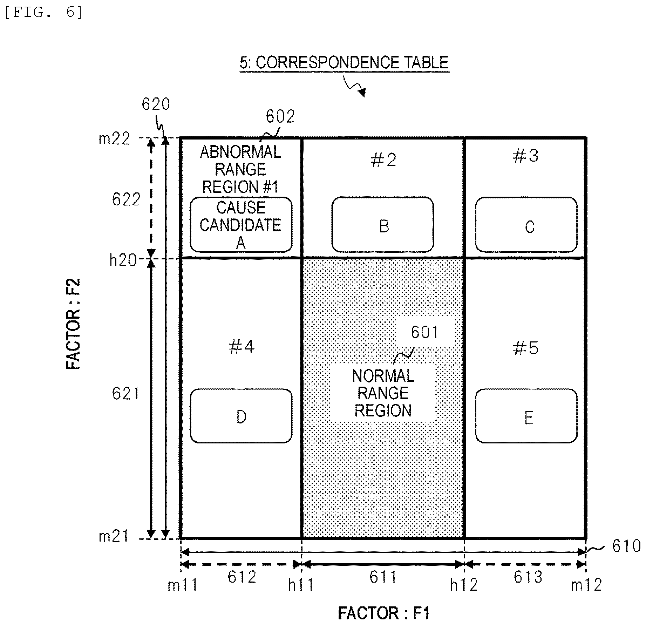

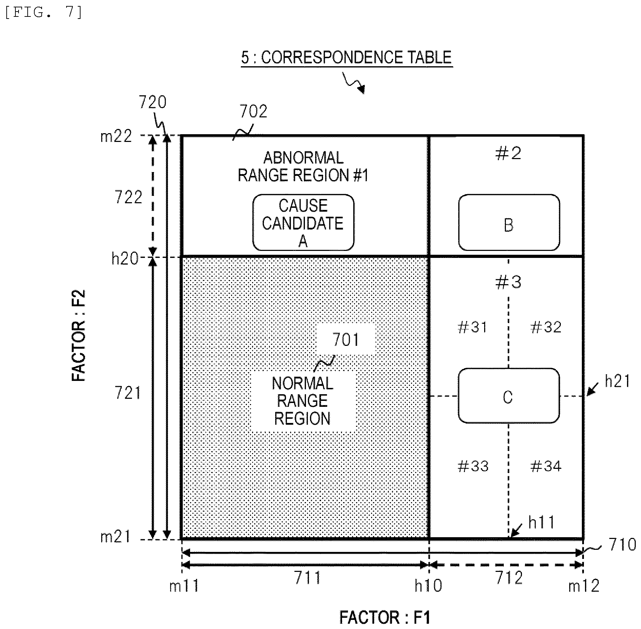

In the example, each of the thresholds of the normal/abnormal ranges of the two factor values includes an upper limit threshold and a lower limit threshold with respect to the normal range as described below (Fig. 5). That is, each of the factor values includes one normal range and two upper and lower abnormal ranges. However, the present invention is not limited to this configuration, and the normal/abnormal ranges of each of the factors may adopt various configurations (for example, Fig. 6 or Fig. 7 described below).

In Step S4, using the factor value of Step S2 and the threshold of the range of Step S3, the data processing device 1 executes the abnormality determination or check the abnormality determination result for the value of each of the factors that is calculated from the measurement result regarding the same specimen as a target sample. When the abnormality determination result data 24 includes the abnormality determination result, the data processing device 1 may check the abnormality determination result. When the abnormality determination result data 24 does not include the abnormality determination result, the data processing device 1 executes the abnormality determination in Step S4.

In Step S5, the data processing device 1 determines the combination of the plurality of factor values (for example, the pair of the factor F1 and the factor F2 as in Fig. 5 described below) based on the determination result of the plurality of factors in Step S4, and determines the range region in the distribution diagram of the correspondence table to which the combination corresponds.

In Step S6, the data processing device 1 checks whether or not the combination of the factor values (a plot of Fig. 9 described below) corresponds to any of the abnormal range regions in the correspondence table as a result of the determination in Step S5. When the combination corresponds to any of the abnormal range regions in Step S6 (Y), the flow proceeds to the following Step S7. When the combination does not correspond to any of the abnormal range regions in Step S6 (N), the flow ends.

In Step S7, the data processing device 1 reads information regarding a cause candidate and a countermeasure associated with the corresponding abnormal range region from the memory 202 based on the correspondence table. In the correspondence table that is set in advance by default by the manufacturer of the automatic analyzer 10, the information regarding a cause candidate and a countermeasure is set to be associated with each of the abnormal range regions.

In the correspondence table, the cause candidate information may be present or absent in each of the abnormal range regions, or the countermeasure information may be present or absent in each of the abnormal range regions.

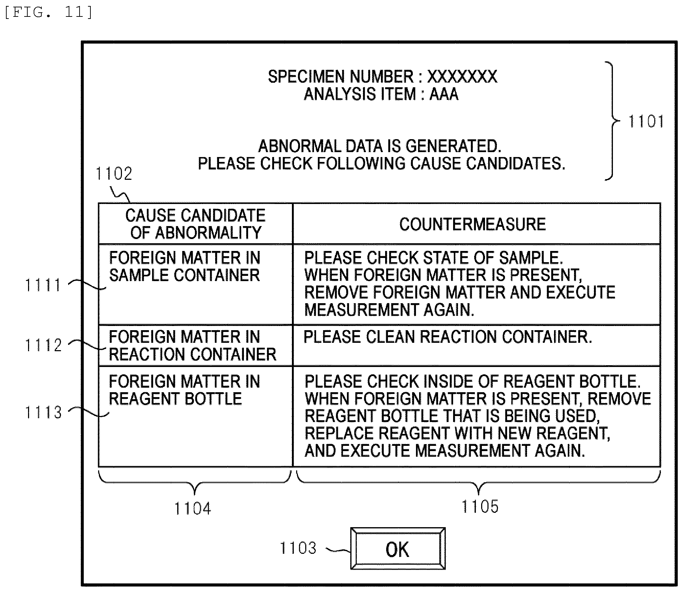

In Step S8, the data processing device 1 generates output information, specifically, data of a screen to the operator using the information regarding the cause candidates and the countermeasures read in Step S7. The screen is, for example, data for display on the display screen of the display device 206 of Fig. 3 and may be in the form of, for example, a Web page. The screen is displayed as in, for example, Fig. 11 described below. The screen includes information for notifying abnormality relating to the measurement of the target sample and information regarding one or more cause candidates and information regarding countermeasures based on the correspondence table. After Step S8, the flow ends. The above-described process is repeated in the same manner whenever the target sample or abnormality occurs. The operator uses the information of the screen in Step S8 as a guide to execute the operation of identifying the cause for the abnormality or taking a countermeasure such as maintenance. As a result, when all of the abnormality causes are removed and the combination of the factor values corresponds to the normal range region of the correspondence table, the data processing device 1 outputs the result showing that the reaction is normal through the screen.

### [Abnormality Detection Function]

The automatic analyzer 10 has a function (also referred to as "abnormality detection function") of determining and detecting abnormality of measurement during or after measurement and analysis. The data processing device 1 has a function of cooperating with the abnormality detection function of the automatic analyzer 10. When the abnormality of the measurement is determined and detected, the control device 101 of Fig. 2 transmits data including abnormality determination result information to the data processing device 1 as an abnormality notification. The data processing device 1 stores the abnormality determination result data 24 based on the reception of the data. As a result, the data processing device 1 executes the process illustrated in Fig. 4 and notifies the occurrence of the abnormality, the urge of the countermeasure, or the like to the operator through the screen.

When the abnormality is detected during the measurement, optionally, the automatic analyzer 10 temporarily stops device operation (for example, the operation of each of the driving mechanisms in Fig. 2) of the automatic analyzer 10 together with the abnormality notification. When the abnormality is resolved after taking a countermeasure such as maintenance work by the operator, the control device 101 of the automatic analyzer 10 restarts the device operation that has been temporarily stopped.

### [Correspondence Table]

Fig. 5 illustrates a concept and a first configuration example of a correspondence table 5. The correspondence table 5 illustrates a two-dimensional table that is configured by the pair of the factors F1 and F2 particularly as the two factors. Here, the horizontal axis represents the factor F1 as the first factor, and the vertical axis represents the factor F2 as the second factor. For each of the factors (F1, F2), predetermined variables (described below) representing characteristics of the reaction in the reaction process data are selected.

Each of the factors has a range of a value that can be adopted. The factor F1 has a range 510 of the factor value. The range 510 is a range from a lower limit value m11 to an upper limit value m12. Each of the factors includes a normal range and abnormal ranges (in other words, ranges outside the normal range). The range 510 includes a normal range 511 indicated by a solid line arrow and two upper and lower abnormal ranges 512 and 513 indicated by broken line arrows. The normal range 511 is a range from a lower limit threshold h11 to an upper limit threshold h12. The lower abnormal range 512 is a range from the lower limit value m11 to the lower limit threshold h11 as a first abnormal range. The upper abnormal range 513 is a range from the upper limit threshold h12 to the upper limit value m12 as a second abnormal range. Likewise, the factor F2 includes a range 520 of the factor value. The range 520 is a range from a lower limit value m21 to an upper limit value m22. The range 520 includes a normal range 521 indicated by a solid line arrow and two upper and lower abnormal ranges 522 and 523 indicated by broken line arrows. The normal range 521 is a range from a lower limit threshold h21 to an upper limit threshold h22. The lower abnormal range 522 is a range from the lower limit value m21 to the lower limit threshold h21. The upper abnormal range 523 is a range from the upper limit threshold h22 to the upper limit value m22. Each of the ranges is defined as, for example, a range of the first threshold or more and less than the second threshold such that the ranges do not overlap each other. During determination of the range region to which one value corresponds, the corresponding one range region is determined.

In the correspondence table 5, by considering the combination of the normal/abnormal ranges of the factors F1 and F2 as a first combination, a plurality of (3 × 3 = 9 in the example of Fig. 5) range regions are configured in the two-dimensional region as illustrated in the drawing. The plurality of range regions include one normal range region 501 and a plurality of (eight) abnormal range regions 502 (represented by #1 to #8) around the normal range region 501. The abnormal range region is a region where at least one of the factor values is in the abnormal range.

In the first configuration example of Fig. 5, since the abnormal range is present on each of the upper and lower sides with respect to the normal ranges (511, 521) of the factors (F1, F2), the eight abnormal range regions in total are configured. In other words, in the example, the normal range of each of the factors (F1, F2) is between the lower limit value and the upper limit value (for example, in the middle) in the entire range and does not overlap the lower limit value or the upper limit value. Therefore, two abnormal ranges are present on opposite sides of the normal range.

The normal range region 501 is a two-dimensional region that is configured by the normal range 511 of the factor F1 and the normal range 521 of the factor F2. The plurality of abnormal range regions 502 are assigned with identification information (for example, #1 to #8) such that each of the abnormal range regions is identifiable. For example, the abnormal range region #1 is a two-dimensional region that is configured by the abnormal range 512 of the factor F1 and the abnormal range 523 of the factor F2. The abnormal range region #2 is a two-dimensional region that is configured by the normal range 511 of the factor F1 and the abnormal range 523 of the factor F2. The abnormal range region #1 corresponds to a case where the values of the two factors (F1, F2) are in the abnormal ranges. The abnormal range region #2 corresponds to a case where the value of the factor F1 is in the normal range and the value of the factor F2 is in the abnormal range.

In addition, in the correspondence table 5, each of the abnormal range regions 502 (#1 to #8) is set to be associated with the information regarding the cause candidate for the abnormality. In the example, cause candidates A, B, ..., G, and H are present as eight cause candidates to be illustrated in the squares of the abnormal range regions. Although not illustrated in the drawing, each of the cause candidates A to H may be set to be associated with the information regarding the countermeasure. The information regarding each of the cause candidates or the countermeasure is assigned with identification information such that the information is identifiable.

In some abnormal range regions, the cause candidates and the like may be different or the same, but either case can be dealt with the correspondence table 5. In the example, the correspondence table is illustrated as the two-dimensional concept that is configured by the two factors. However, the correspondence table is not limited to this example and can also be configured as a three- or higher dimensional concept that is configured by three or more factors.

Here, the important point is as follows. In an abnormality determination method in an automatic analyzer in the related art, for example, values of predetermined variables representing characteristics of a reaction process are compared to a condition of a range that is configured by a predetermined threshold to determine whether or not the reaction is normal or abnormal in many cases. That is, in the related art, roughly, only two cases including a case where each of the predetermined variables is in a normal range and a case where each of the predetermined variables is not in the normal range (in the abnormal range) are considered. In the related art, when it is determined that abnormality of measurement occurs based on the fact that the variable value is in the abnormal range, a plurality of abnormality cause candidates are present and the abnormality cause is unclear. Therefore, there are many cases where it is difficult for the operator to identify the abnormality cause or time and effort are required for the identification.

On the other hand, in the first embodiment, as in the correspondence table 5 described above, for example, the combination of the two factors is considered, and the plurality of abnormal range regions 502 are specifically considered around the normal range region 501. In the first embodiment, the cause candidate and the like relating to the abnormality are considered in each of the abnormal range regions 502. Even a case where the abnormal range regions 502 have different cause candidates and the like can be dealt with the association in the correspondence table 5. As a result, in the first embodiment, the cause candidate or the countermeasure corresponding to the abnormal range region can be presented. Therefore, the identification of the cause or the like by the operator is easier than that in the related art.

Fig. 6 illustrates a second configuration example of the correspondence table 5 as another example. In the second configuration example of the correspondence table 5 of Fig. 6, a normal range of one factor overlaps a lower limit value of a range. Specifically, in the correspondence table 5, for the factor F1, as in Fig. 5, a range 610 includes an abnormal range 612, a normal range 611, and an abnormal range 613. On the other hand, for the factor F2, a range 620 includes a normal range 621 and an abnormal range 622. The normal range 621 overlaps the lower limit value m21 of the range 620 and is a range from the lower limit value m21 to a threshold h20. The abnormal range 622 is a range from the threshold h20 to the upper limit value m22. As a result, the correspondence table 5 includes one normal range region 601 and five abnormal range regions 602 (#1 to #5) around the normal range region 601. The abnormal range regions 602 are set to be associated with cause candidates (A to E), respectively. For example, the abnormal range region #4 is configured by the abnormal range 612 of the factor F1 and the normal range 621 of the factor F2.

Fig. 7 illustrates a third configuration example of the correspondence table 5 as still another example. In the third configuration example of the correspondence table 5 of Fig. 7, a normal range of each of two factors overlaps a lower limit value of a range. Specifically, in the correspondence table 5, for the factor F1, a range 710 includes a normal range 711 and an abnormal range 712 such that the ranges are divided by a threshold h10. For the factor F2, a range 720 includes a normal range 721 and an abnormal range 722 such that the ranges are divided by the threshold h20. As a result, the correspondence table 5 includes one normal range region 701 and three abnormal range regions 702 (#1 to #3) around the normal range region 701. The abnormal range regions 702 are set to be associated with cause candidates (A to C), respectively.

Further, in a modification example, in the correspondence table, as indicated as a region of a broken line in Fig. 7, one abnormal range region may be further divided into a plurality of abnormal range regions as a subordinate concept. Each of the divided abnormal range regions may be associated with information regarding a cause candidate or the like. For example, the abnormal range region #3 is further divided into four abnormal range regions (#31 to #34). The abnormal range 712 of the factor F1 is divided using the threshold h11. The normal range 721 of the factor F2 is divided using the threshold h21. For example, the abnormal range region #31 is configured by an abnormal range from the threshold h10 to the threshold h11 of the factor F1 and a normal range from the threshold h21 to the threshold h20 of the factor F2.

In the first embodiment, the configuration of the correspondence table 5 is set in the data processing device 1 (the control program 21, the setting information 22, the correspondence table data 26, and the like in Fig. 3) in advance as design items and a fixed value. The correspondence table is set by default in advance before product delivery by a business operator such as the manufacturer of the automatic analyzer 10. That is, the information regarding the factors, the value ranges, the threshold, the cause candidate, and the countermeasure associated with each of the abnormal range regions, and the like that are used in the correspondence table are preset. The correspondence table can be set without being limited to fixed values. In modification examples described below, the information of the correspondence table can be set to be variable on the device by the operator.

The first combination of the plurality of factors is multi-dimensional information. When this information is visualized on the screen for the operator, two-dimensional information may be generated and displayed for easy understanding. That is, as in Fig. 5 or the like, for example, the data processing device 1 may generate the two-dimensional distribution diagram to display the distribution diagram for each pair of two factors that are focused on and selected.

When a plurality of factors that can be used for identifying the abnormality cause are present, a plurality of correspondence tables corresponding to combinations of factors to be used may be prepared. For example, when factors F1, F2, and F3 are present, a first correspondence table corresponding to a combination of the factors F1 and F2, a second correspondence table corresponding to a combination of the factors F3 and F1, and the like are prepared. The operator may select one to be used from the plurality of factors or the plurality of correspondence tables.

### [Measured value]

The measurement data 23 (Fig. 3) from the automatic analyzer 10 for a clinical test having a function such as biochemical analysis includes a measured value at each time point. This "measured value" refers to a value having a predetermined unit, for example, a concentration or an activity value calculated based on absorbance data, and can be one factor.

### [Reaction Process Data]

The reaction process data in the measurement data 23 from the automatic analyzer 10 will be described. The reaction process data represents a time change in absorbance or the like in a period of time from the start to the end of the measurement of the sample. The predetermined variables can be calculated based on the reaction process data and are variables representing characteristics of the reaction process.

Fig. 8 illustrates an example of reaction process data 8. The reaction process data 8 is data representing a time change in absorbance (represented by reference numeral A; the unit is the dimensionless amount) of the sample. In the graph of Fig. 8, the horizontal axis represents the time (the measurement time; represented by reference numeral T; the unit is [min]), and the vertical axis represents the absorbance A. The automatic analyzer 10 measures the absorbance A of the sample in the reaction container, and calculates the concentration or the like of a component material of the sample as the measured value from the absorbance A. In the graph, a plot indicated by black points represents the value of the absorbance A measured at each time point. For example, in a period of time T1 from about 0 minutes to five minutes, the absorbance A is a substantially given value a1. In a period of time T2 (a plot from a time point t1 to a time point t2) from about 5 minutes to 10 minutes, the absorbance A schematically linearly increases from a value a2 to a value a3.

A line 801 is an asymptotic line, in other words, an approximation line regarding a distribution of the absorbance A that is generated based on the plot of the absorbance A in the period of time T2, and has a positive gradient. The gradient (represented by G) of the asymptotic line is a value that varies depending on the sample, the analysis item, the reaction during the measurement, or the like, and represents a characteristic of the reaction. A difference 802 indicated by an arrow is a difference between a value 803 of the absorbance A at one time point tx and the line 801. When the difference 802 is large, that is, when a deviation from the asymptotic line is large, abnormality of the measurement is suspicious.

### [Factor]

Next, the factor will be described. The factor is configured by a measured value or a variable in the reaction process data 8 described above. In the example of the first embodiment, the variable is used as the factor. The factors used in the correspondence table 5 are set in advance by default in the first embodiment as described above.

Fig. 9 illustrates a distribution diagram that is configured by the combination of the two factors (F1, F2) representing characteristics of the reaction of the reaction process data 8 illustrated in Fig. 8. In the example of the first embodiment, the factor F1 is the gradient G of the asymptotic line (line 801) of Fig. 8, and the factor F2 is a mean square error (represented by reference numeral E).

In the distribution diagram of Fig. 9, the horizontal axis represents the gradient G of the asymptotic line as the factor F1, and the vertical axis represents the mean square error (E) as the factor F2. The mean square error (E) is a value obtained by squaring and adding the differences between the asymptotic line and the absorbance A at the respective time points, for example, the difference 802 in the period of time T2 having the plot of the absorbance A corresponding to the asymptotic line (line 801). The mean square error (E) is represented by, for example, the following expression. The plot of the absorbance A at time point t is represented by A(t). The difference such as the difference 802 is represented by D(t). The respective time points in the period of time T2 are a plurality of (n) time points such as t = t1 to t2. E = ΣD(t)². The arithmetic operation Σ is the addition at t = t1 to t2. The distribution diagram of the combination of the factor values as in Fig. 9 specifically corresponds to the first combination of the ranges of the factor values as a part of the correspondence table 5 as in Fig. 5.

In the example, both of the factors (F1, F2) of the distribution diagram can adopt a value of 0. A range 910 of the factor F1 is a range from a minimum value m11 to a maximum value m12, and the minimum value m11 is 0. A range 920 of the factor F2 is a range from a minimum value m21 to a maximum value m22, and the minimum value m21 is 0. In addition, in the example, the normal range of the factor F1 and the normal range of the factor F2 have a predetermined relationship, and a normal range region 901 is configured by the combination of the factor values. The normal range region 901 is not limited to a simple rectangular region as in Fig. 5. In the example, the normal range region 901 is a belt-shaped region that includes the lower limit values (0) and extends obliquely from the lower left side to the upper right side. In other words, the normal range region 901 is defined by a predetermined expression as a region where the value of the factor F1 and the value of the factor F2 have a predetermined relationship such as a linear shape. The normal range region can adopt any shape corresponding to a precondition or the like as described above.

Therefore, in the example, the factor value distribution diagram includes the normal range region 901 and, for example, three abnormal range regions 902 (#1 to #3) around the normal range region 901. The range 910 of the factor F1 includes, for example, values f11, f12, f13, and f14, and the range 920 of the factor F2 includes, for example, values f21, f22, f23, and f24. For example, when the factor F1 satisfies m11 = 0, the range of the factor F2 from 0 to the value f21 is the normal range region, and the range of the value f21 or more is the abnormal range region #1. When the factor F1 is the value f11, the range of the factor F2 from 0 to the value f23 is the normal range region, and the range of the value f23 or more is the abnormal range region #1. When the factor F1 is the value f12, the range of the factor F2 from 0 to the value f24 is the abnormal range region #3, and the range from the value f24 to the upper limit value m22 is the normal range region. When the factor F1 is the value f14, the range of the factor F2 from 0 to the value f22 is the abnormal range region #3, and the range from the value f22 to the upper limit value m22 is the normal range region. When the factor F1 is the value f13, the range of the factor F2 from 0 to the value f22 is the abnormal range region #3, and the range from the value f22 to the upper limit value m22 is the abnormal range region #2.

In the example, the abnormal range region 902 that is present on the lower right side with respect to the normal range region 901 corresponds to the value f22 (corresponding to the threshold) and is divided into two upper and lower abnormal range regions (#2, #3). In addition, in the distribution diagram, the threshold that divides the normal/abnormal ranges is configured by, for example, lines 931 and 932.

In the distribution diagram, the plot represented by black points is the plot that is generated by the pair of the value of the factor F1 and the value of the factor F2 based on the measurement data 23, in other words, the combination of the factor values. For example, in the normal range region 901, a distribution of a plot group 903 is present in a lower portion close to the lower right (0). The plot group 903 corresponds to a case where the measurement is determined to be normal. In addition, in the example, one plot 904 that is present outside the plot group 903 is provided. The plot 904 is present in the abnormal range region #1. The plot 904 corresponds to a case where the measurement is determined to be abnormal.

### [Example of Abnormality and Cause Candidate]

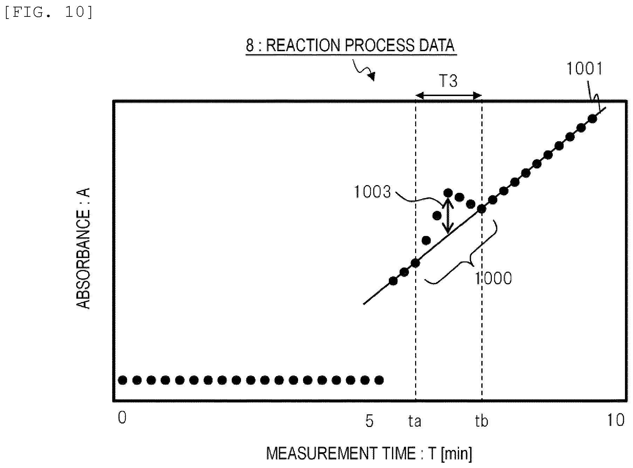

Fig. 10 illustrates an example of the reaction process data 8 including the data of the plot 904 that is distributed in the abnormal range region #1 in the factor value distribution diagram of Fig. 9. In the reaction process data 8 of Fig. 10, the gradient (G) of the asymptotic line as the factor F1 is in the normal range (0 to f11) as illustrated in Fig. 9, but the mean square error (E) as the factor F2 is a value more than the normal range (about 0 to f22).

In the reaction process data 8 of Fig. 10, only in a range 1000 (a period of time T3 in the vicinity of time points ta to tb), the absorbance A deviates from a line 1001 as an asymptotic line. That is, in the range 1000, a difference 1003 between the asymptotic line and the absorbance A is large. In this case, in a region other than the range 1000, it is considered that the reaction occurs normally. The period of time T3 corresponding to the range 1000 is considered to correspond to the abnormal state. Therefore, in the period of time T3 of the range 1000, one abnormality cause candidate is presumed to be that an optical axis of the photometer (a part of the optical measurement mechanism 16 of Fig. 2) may be temporarily blocked by foreign matter.

### [Output]

As an output example of the information based on the correspondence table 5 by the data processing device 1, a display example of a screen will be described. The data processing device 1 provides a screen including specific information or a graphical user interface (GUI).

Fig. 11 illustrates a screen example when information regarding the cause candidate or the countermeasure associated with the abnormal range region #1 is notified to the operator when abnormality corresponding to the abnormal range region #1 in the correspondence table 5 having the factor value distribution diagram illustrated in Fig. 9 is detected. The screen of Fig. 11 includes, for example, a message 1101, a table 1102, and a button 1103. The message 1101 includes information regarding the target sample, the analysis item, or the like and information to be notified to the operator (for example, "Abnormal data is generated. Please check the following cause candidates and the like.") . The table 1102 includes a "Cause Candidate of Abnormality" item 1104 and a "Countermeasure" item 1105. In the "Cause Candidate of Abnormality" item 1104, information regarding one or more cause candidates associated with the corresponding abnormal range region (the abnormal range region #1 of Fig. 9) of the correspondence table 5 (for example, the distribution diagram of Fig. 9) is displayed. In the example, three cause candidates (1111, 1112, 1113) associated with the abnormal range region #1 are provided and are displayed to be divided by rows in the "Cause Candidate of Abnormality" item 1104. In the "Countermeasure" item 1105, information regarding the countermeasures associated with the cause candidates on the left side is displayed.

In the example, for one abnormality (the plot 904 of Fig. 9, the range 1000 of Fig. 10), the three cause candidates (1111 to 1113) are extracted and presented. The plurality of cause candidates may be assigned with identification information and displayed. In the example, the plurality (three) of cause candidates do not have any order relation or the like but are displayed to be longitudinally arranged. For example, the cause candidate 1111 is "Foreign Matter in Sample Container", the cause candidate 1112 is "Foreign Matter in Reaction Container", and the cause candidate 1113 is "Foreign Matter in Reagent Bottle". In addition, the countermeasure against the cause candidate 1111 is "Please check the state of the sample. When foreign matter is present, remove the foreign matter and execute the measurement again." The countermeasure against the cause candidate 1112 is "Please clean the reaction container." The countermeasure against the cause candidate 1113 is "Please check the inside of the reagent bottle. When foreign matter is present, remove the reagent bottle that is being used, replace the reagent with a new reagent, and execute the measurement again."

The button 1103 is, for example, an OK button. The operator sees and checks the information on the screen, grasps the cause candidates or the countermeasures, identifies the cause, and executes the countermeasure. As a result, the abnormal state is resolved, the screen displays that the reaction is normal, and the operator presses the button 1103 to end the screen.

In addition, in the table 1102, for example, a check item may be provided in each of the rows corresponding to the cause candidates such that, when the operator checks or executes each of the cause candidates or executes each of the countermeasures, a check can be marked in the corresponding check item. In addition, for the plurality of cause candidates and the like of the abnormal range region in the correspondence table 5, an order, a priority, or the like representing the degree of possibility may be defined. In this case, in the table 1102, the plurality of cause candidates and the like may be assigned with numbers and displayed in accordance with the order, the priority, or the like.

The screen is not limited to the screen example of Fig. 11 and can adopt another screen example. The data processing device 1 may display an image of the correspondence table 5 as illustrated in Fig. 5 on the screen, or may display an image of the factor value distribution diagram as illustrated in Fig. 9. In other words, the factor value distribution diagram is an image that is visualized by the first combination of the normal/abnormal ranges of the plurality of factors. When the data processing device 1 displays the distribution diagram as illustrated in Fig. 9, the plot of the combination of the factor values corresponding to the currently detected abnormality and the abnormal range region to which the plot corresponds may be highlighted and displayed, for example, by color coding.

In addition, the data device device 1 may display the graph of the reaction process data 8 as illustrated in Fig. 8. The data device device 1 may display the graph of the calculated factor values, for example, the graph where the horizontal axis represents the time and the vertical axis represents the factor value on the screen. In addition, when the correspondence table 5 or the distribution diagram is displayed on the screen, the data processing device 1 may display the threshold that divides the normal/abnormal ranges, the expression corresponding to the threshold, or the like such that the threshold or the expression can be checked. In addition, the threshold or the expression may be set to be variable by the user such as the manufacturer. For example, when the distribution diagram of Fig. 9 is displayed on the screen, the data device device 1 displays the value f22 corresponding to the threshold that divides the abnormal range regions #2 and #3 and changes the value f22 or a line corresponding to the value f22 to update the setting by the operation of the user. As a result, adjustment relating to the plurality of abnormal range regions can be executed.

### [Effects and the like]

As described above, in the automatic analyzer and the data processing device according to the first embodiment, information regarding a cause candidate or the like of abnormality of measurement can be presented based on the specific correspondence table 5. Therefore, the automatic analyzer and the data processing device can support the operator to rapidly or easily identify a cause for abnormality of measurement and to improve the abnormality. In the first embodiment, even when a plurality of cause candidates for abnormality of measurement are present, information corresponding to the abnormal range region to which the abnormal corresponds is presented. Therefore, the operator can rapidly and easily identify the cause. As a result, the troubleshooting time can be reduced. In particular, even when the operator is a person who does not have much experience relating to automatic analysis, the support of the output information by the data processing device can contribute to the learning of the operation or the knowledge or the skill improvement. In the related art, only the abnormality determination on whether or not one factor value is in the normal range is made. In the first embodiment, by combining the plurality of factor values, the plurality of abnormal range regions may be provided outside the normal range region such that a cause candidate or the like corresponding to each of the abnormal range regions can be presented.

In the first embodiment, by using the pair of the gradient (G) of the asymptotic line and the mean square error (E) in the reaction process data (Fig. 9) as the combination of the factors, characteristics relating to abnormality of measurement can be grasped. In this case, in the correspondence table 5 including the factor value distribution diagram, for example, as in Fig. 9, the plurality of abnormal range regions 902 (#1 to #3) outside the normal range region 901 can be configured, and the cause candidates corresponding to the abnormal range regions 902 can be grasped to be distinguishable from each other.

The following modification examples of the first embodiment can also be adopted. In the data processing device 1 according to the modification example, a new correspondence table 5 (in particular the factor value distribution diagram as illustrated in Fig. 9) based on input data may be generated through schematically the same flow as the flow of Fig. 4. The data processing device 1 displays the generated correspondence table 5 on the screen, and the operator or the manufacturer person can input and set the information regarding a cause candidate or a countermeasure for each of the items of the correspondence table 5. For example, when the correspondence table illustrated in Fig. 5 is generated, the data processing device 1 inputs the information (for example, m11, h11, h12, m12) that defines the normal/abnormal ranges of the factor F1 and the information (for example, m21, h21, h22, m22) that defines the normal/abnormal ranges of the factor F2 based on the user input. By processing the information, the data processing device 1 generates the distribution diagram including the normal range region and the plurality of abnormal range regions as illustrated in Fig. 5. The data processing device 1 sets the information regarding a cause candidate or a countermeasure to be associated with each of the ranges of the distribution diagram based on the user input.

### [First Modification Example]

In a first modification example of the first embodiment, the data processing device 1 has a function capable of setting the information of the correspondence table 5 to be variable by the operator. The operator can operate the input device 205 illustrated in Fig. 3 to input information regarding a new factor, an abnormality cause candidate, a countermeasure, or the like and to set the information to the correspondence table data 26 while seeing the screen of the display device 206. That is, for example, the information regarding an abnormality cause candidate or a countermeasure in the correspondence table 5 can be added or modified by the operator.

Note that it is desirable to distinguish between the information of the correspondence table that is set in advance by default by the manufacturer and the information that is added, modified, etc. by the operator (in other words, user input information) without any confusion. Accordingly, the data processing device 1 manages the correspondence table data 26 and displays the information of the correspondence table on the screen to distinguish between the information. The data processing device 1 stores and manages, for example, information regarding an input source (for example, whether to be the manufacturer or the operator) for the information regarding each of the items of the correspondence table. For example, the data processing device 1 stores the information of the default correspondence table without permitting deletion or change. When information of the correspondence table that is updated by the manufacturer is present, the data processing device 1 may store the updated information.

The data processing device 1 separately stores and manages the user input information such as the addition or modification, etc. by the operator in association with the default correspondence table. When the information of the correspondence table is displayed on the screen, the data processing device 1 displays the default information and the updated information by the operator to distinguish between the information, for example, by color-coding the information, by displaying the information in different fields, or by displaying information regarding the input source together. The data processing device 1 may display any one of the default information or the updated information by the operator on the screen in accordance with the selection operation by the operator. Alternatively, both of the default information and the updated information by the operator may be displayed in parallel on the screen.

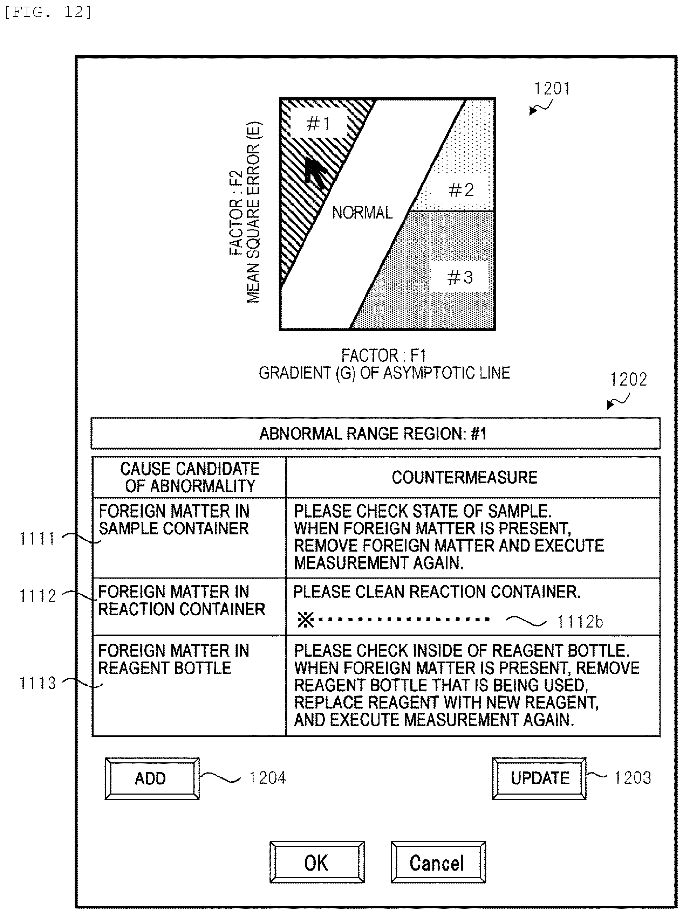

Fig. 12 illustrates a screen example that is provided by the data processing device 1 in the first modification example. The screen of Fig. 12 includes a correspondence table 1201 and a table 1202. In the correspondence table 1201, for example, the portion of the factor value distribution diagram as illustrated in Fig. 9 is displayed, and the normal range region and the plurality of abnormal range regions are displayed to be distinguishable by identification information, color coding, or the like. The information regarding a threshold of a range or the like may be displayed together with the correspondence table 1201. The operator may select the range region from the correspondence table 1201. In response to the operation, information associated with the selected range region is displayed in the table 1202.

The table 1102 of Fig. 11 is fixed and cannot be modified by the operator. On the other hand, the table 1202 of Fig. 12 has a GUI where the information can be added, modified, etc. by the operator. In the table 1202, the operator can add characters, an image, or the like in each of the items. For example, in the row of the cause candidate 1112, characters 1112b such as an additional remark or an URL are added to the item of the countermeasure by the operator. The screen has an update button 1203, when the information of the table 1202 is updated to the input state, the operator presses the update button 1203 (which may be the OK button). As a result, the updated correspondence table 5 is stored. In addition, the screen has an addition button 1204. When information regarding a cause candidate and a countermeasure is added, the operator presses the addition button 1204. As a result, a new row is added to the table 1202. The operator inputs information regarding a cause candidate and a countermeasure to the row and presses the update button 1203 for the update.

In the first modification example, the operator can freely set the association between the abnormal range region of the factor and the information regarding a cause candidate and a countermeasure. For example, the following advantages can be achieved by the configuration. The operator does not need to wait for the update of the correspondence table by the manufacturer and can easily update the correspondence table to the latest information in accordance with the status or the history of the use of the automatic analyzer 10. The operator can describe his or her own idea, a memo, or the like in the correspondence table. When one specific automatic analyzer 10 is in a unique state, for example, when a specific phenomenon occurs in a facility where the automatic analyzer 10 is provided, the operator can describe information to reflect the information on the correspondence table. Examples of the phenomenon include mixing of foreign matter derived from a specimen caused by insufficient preliminary preparation (for example, centrifugal separation) of the specimen and an abnormal reaction process caused by insufficient maintenance. As a result, the operator can easily execute the operation.

### [Second Modification Example]

The factors used in the correspondence table 5 are not limited to the above-described examples. Examples of other factors are as follows.

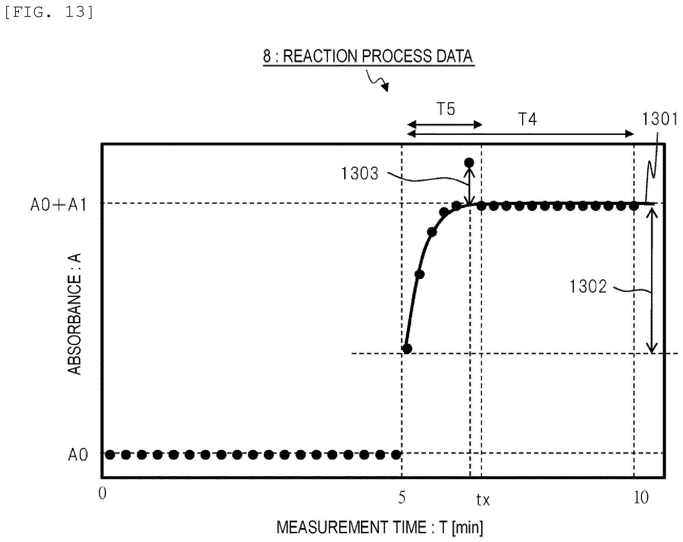

Fig. 13 illustrates an example of the reaction process data 8 in the second modification example. The example is an example of the reaction process data 8 in an end point method (described in PTL 1) used for measurement and analysis of a biological sample. In this case, in the reaction process data 8, a time change in the absorbance A is not the line as in Fig. 8 or the like and is a curve as in an approximate expression 1301 in a period of time T4. In the curve of the approximate expression 1301, the absorbance A increases with a sharp gradient in a period of time T5 from 5 minutes to the time point tx, the gradient gradually becomes gentle, and the gradient becomes a line of 0 in a period of time after the time point tx. A difference 1302 is a difference in the absorbance A from the addition of a second reagent (about 5 minutes) to the completion of the reaction (about 10 minutes). A difference 1303 is a difference between the absorbance A at a given time point and the approximate expression 1301 as in the difference 802 of Fig. 8.

As the approximate expression 1301, a logistic curve can be used, for example, as described in PTL 1. In this curve, when the reaction time elapses to some extent, the absorbance A is approximated to a given value (A0 + A1). The approximate expression 1301 can be calculated using, for example, a least squares method and is represented by A = A0 + A1 (1-e^{-kt}). A0 represents the reaction start time point absorbance, A1 represents the final reaction absorbance, k represents the reaction rate constant, and t represents the time.

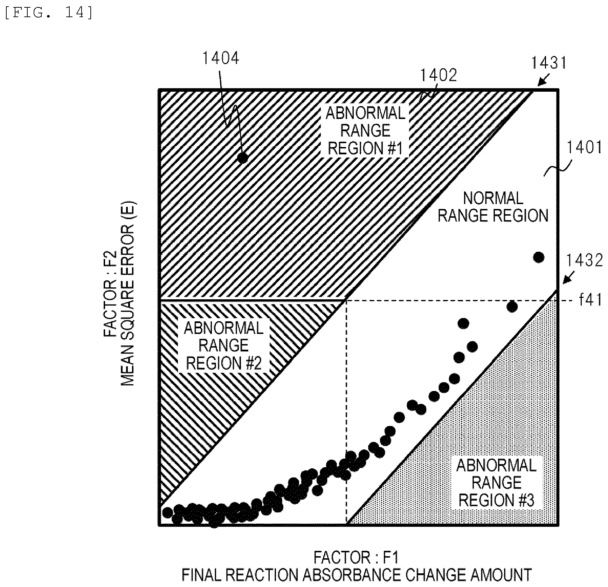

Fig. 14 illustrates an example of the factor value distribution diagram corresponding to the reaction process data 8 of Fig. 13. As in the reaction process data 8 of Fig. 13, as the factor F1, a final reaction absorbance change amount based on the approximate expression 1301 (in other words, the approximated curve) can be used. The final reaction absorbance change amount is a change amount per period of time in the approximate expression 1301, in other words, the gradient at each time point of the approximated curve. The final reaction absorbance change amount is the change amount particularly in a portion approximated to the given value (A0 + A1) in the period of time T5 of Fig. 13. As the factor F2, the mean square error (E) can be used as described above. In this combination of the factors, a distribution diagram illustrated Fig. 14 can be generated. The distribution diagram of Fig. 14 includes an oblique belt-shaped normal range region 1401 and three abnormal range regions 1402 (#1 to #3) around the normal range region 1401. The two abnormal range regions #1 and #2 on the upper left side of the normal range region 1401 include the upper abnormal range region #1 and the lower abnormal range region #2 based on a value f41 as a threshold. The one abnormal range region #3 is provided on the lower right side of the normal range region 1401. The threshold of the normal/abnormal ranges is represented by lines 1431 and 1432 as in the case of Fig. 9. Each of the abnormal range regions 1402 can be associated with a cause candidate or the like. A plot 1404 that is a combination of the factor values is an example of abnormality corresponding to the abnormal range region #1.

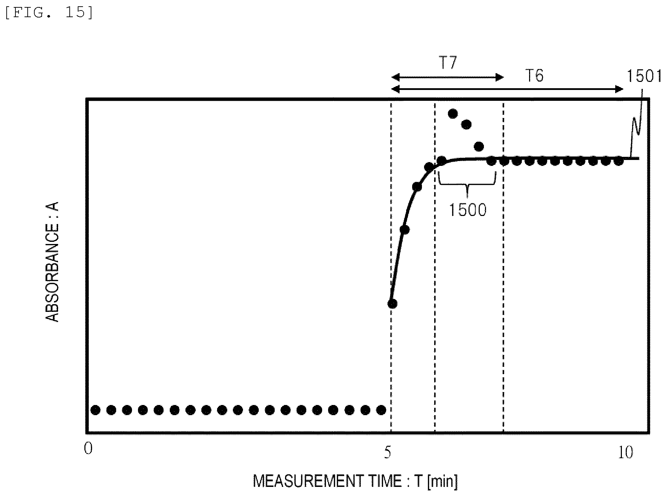

Fig. 15 illustrates an example of the reaction process data 8 in the data that corresponds to the plot 1404 corresponding to the abnormal range region #1 in the factor value distribution diagram of Fig. 14. A time change in the absorbance A in a period of time T6 is represented by a curve of an approximate expression 1501 as in the example of Fig. 13. In a period of time T7, a range 1500 corresponds to a portion of a final reaction approximated to a given value and is a range of a plot that deviates from the approximate expression 1501. The range 1500 is similar to the example of the range 1000 of Fig. 10 and corresponds to abnormality.

In the correspondence table 5 including the reaction process data 8 and the combination of the factors as illustrated in Figs. 13 to 15, for example, the same information regarding the cause candidates and the countermeasures as that of Fig. 11 can be output on the screen illustrated in Fig. 11. As described above, the approximate expression relating to the reaction process is not limited to a line and can be a curve or the like. By using the variables such as the gradient configuring the approximate expression as the factor, the characteristics of the reaction can be grasped well, and plural kinds of abnormality causes can be distinguished and grasped.

### [Third Modification Example]

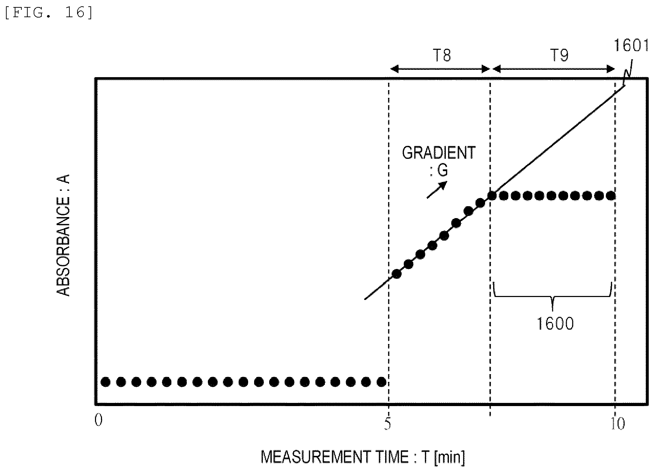

Fig. 16 illustrates an example of data including an abnormal range 1600 as an example of the reaction process data 8 in a third modification example. In a period of time from about 5 minutes to 10 minutes, a time change in the absorbance A is represented by an asymptotic line 1601 and has the gradient (G). In a period of time T8, a plot of the absorbance A has a schematically given gradient. The range 1600 in a period of time T9 is a range of a plot that deviates from the asymptotic line 1601 and corresponds to abnormality.

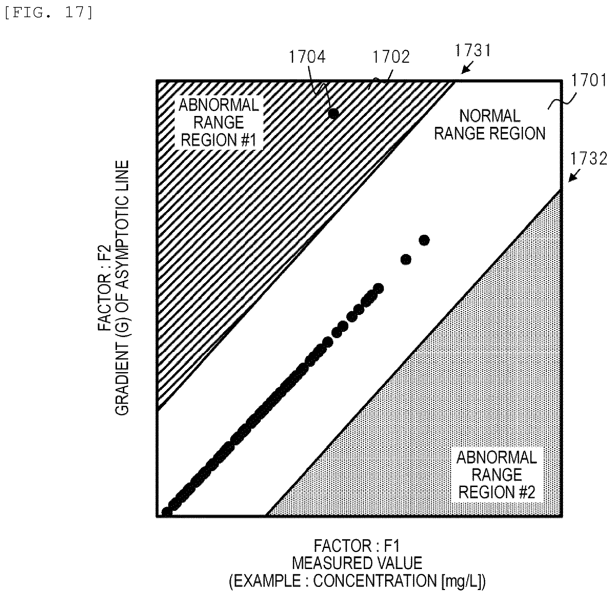

Fig. 17 illustrates an example of the factor value distribution diagram in the third modification example. In the example, in the combination of the factors, the factor F1 is a measured value, and the factor F2 is the gradient (G) of the asymptotic line as in Fig. 8. The measured value as the factor F1 is, for example, the concentration of a component material calculated based on the absorbance. The unit of the concentration is, for example, [mg/L].

In the distribution diagram of Fig. 17, a normal range region 1701 and two abnormal range regions 1702 (#1, #2) are configured by the combination of the values of the factors F1 and F2. The abnormal range region #1 is provided on the upper left side of the normal range region 1701, and the abnormal range region #2 is provided on the lower right side of the normal range region 1701. The threshold of the normal/abnormal ranges is represented by lines 1731 and 1732. In the normal range region 1701, the plot of Fig. 16 is linearly distributed. A plot 1704 of the combination of the factors F1 and F2 is in the abnormal range region #1 and has a relationship with the data of the range 1600 of Fig. 16.

When the abnormality is in the abnormal range region #1 based on the measured value of the measurement data 23, the data processing device 1 outputs the information regarding a cause candidate or the like associated with the abnormal range region #1 to the screen based on the correspondence table 5.

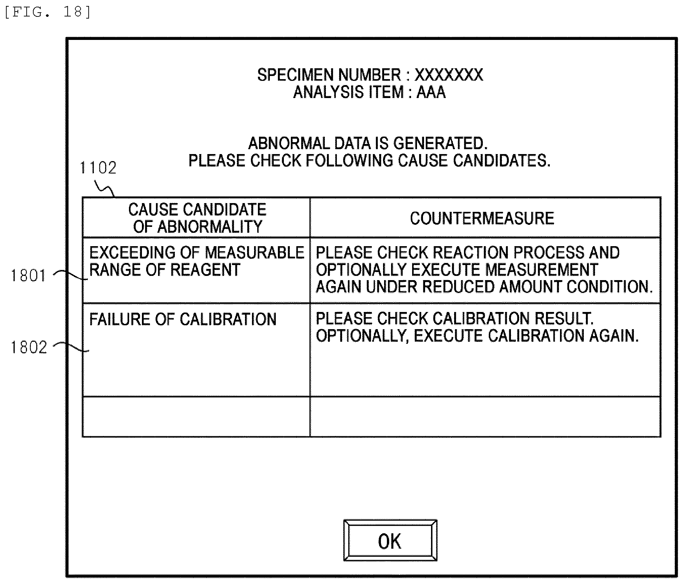

Fig. 18 illustrates a screen example that is output when the plot 1704 of Fig. 17 is abnormal. The configuration of the screen of Fig. 18 is the same as Fig. 11. In the example, two cause candidates are displayed in the "Cause Candidate of Abnormality" item of the table 1102. A cause candidate 1801 is "Exceeding of Measurable Range of Reagent." A cause candidate 1802 is "Failure of Calibration." Fig. 17 illustrates the reaction process that is observed when the cause candidate 1801 "Exceeding of Measurable Range of Reagent" occurs. In Fig. 17, the abnormality does not correspond to the cause candidate 1802 "Failure of Calibration", but when a liquid having a high absorbance is erroneously measured in blank measurement of calibration, a plot may be generated in the abnormal range region #1 as in the example of the plot 1704. The cause candidate 1801 and the cause candidate 1802 can be distinguished from each other based on, for example, whether or not an alarm is output. In the "Countermeasure" item, a countermeasure associated with each of the cause candidates is displayed. The countermeasure against the cause candidate 1801 is "Please check the reaction process and optionally execute measurement again under a reduced amount condition." The countermeasure against the cause candidate 1802 is "Please check the calibration result. Optionally, execute calibration again."

As described above, even when the measured value such as a concentration or an activity value is used as the factor, the characteristics of the reaction can be grasped well, and plural kinds of abnormalities and causes can be distinguished and grasped.

### Reference Signs List

1: data processing device
5: correspondence table
8: reaction process data
10: automatic analyzer
F1, F2: factor
901: normal range region
902 (#1 to #3): abnormal range region

## Claims

1. A data processing device (1) configured to execute the following processes of:
referring (S1) to data regarding measurement of a sample by an automatic analyzer (10);
determining (S6) an abnormal range region; and
outputting (S8) information regarding an abnormality cause candidate to a user,
**characterized in that** the data processing device (1) is configured to execute the following further processes:
calculating (S2) a combination of values of a plurality of factors based on reaction process data among the data regarding measurement of the sample, the factors being predetermined variables or measured values representing characteristics of a reaction;
referring (S5) to a correspondence table (5) including a distribution diagram and information regarding abnormality cause candidates, the distribution diagram including a normal range region and a plurality of abnormal range regions that are generated by a combination of normal/abnormal ranges of the plurality of factors, and the abnormality cause candidates being associated with each of the abnormal range regions, wherein the distribution diagram includes the normal range region (501, 601, 701, 901), a first abnormal range region (502, 602, 702, 902), a second abnormal range region (502, 602, 702, 902), and a third abnormal range region (502, 602, 702, 902) that are generated by at least a combination of a normal range (511, 611, 711) and an abnormal range (512, 513, 612, 613, 712) of a first factor and a normal range (521, 621, 721) and an abnormal range (522, 523, 622, 722) of a second factor, the normal range region (501, 601, 701, 901) being configured by the normal range (511, 611, 711) of the first factor and the normal range (521, 621, 721) of the second factor, the first abnormal range region (502, 602, 702, 902) being configured by the normal range (511, 611, 711) of the first factor and the abnormal range (522, 523, 622, 722) of the second factor, the second abnormal range region (502, 602, 702, 902) being configured by the abnormal range (512, 513, 612, 613, 712) of the first factor and the normal range (521, 621, 721) of the second factor, and the third abnormal range region (502, 602, 702, 902) being configured by the abnormal range (512, 513, 612, 613, 712) of the first factor and the abnormal range (522, 523, 622, 722) of the second factor, wherein
determining (S6) an abnormal range region is determining (S6) the abnormal range region (502, 602, 702, 902) in the correspondence table (5) to which the combination of the values of the plurality of factors corresponds; and
outputting (S8) information regarding an abnormality cause candidate is outputting (S8) the information regarding the abnormality cause candidate associated with the corresponding abnormal range region (502, 602, 702, 902).

2. The data processing device according to Claim 1,
wherein the correspondence table **(5)** is set such that each of the abnormal range regions or each of the abnormality cause candidates is associated with information regarding a countermeasure.

3. The data processing device according to Claim 1,
wherein the correspondence table (5) is set in advance as default setting information by a business operator of the automatic analyzer.

4. The data processing device according to Claim 1,
wherein the correspondence table (5) is updated by displaying information regarding the correspondence table on a screen and adding or modifying the information regarding the abnormality cause candidate based on an operation of the user on the screen.

5. The data processing device according to Claim 1,
wherein the reaction process data is data representing a time change in absorbance, and
as the variable that is one of the factors, a variable configuring an approximate expression of the reaction process data is used.

6. The data processing device according to Claim 1,
wherein the reaction process data is data representing a time change in absorbance, and
as the variable that is one of the factors, a mean square error regarding a difference between an approximate expression and an absorbance in the reaction process data is used.

7. The data processing device according to Claim 1,
wherein the reaction process data is data representing a time change in absorbance, and
as the measured value that is one of the factors, a concentration or an activity value of a component of the sample is used.

8. The data processing device according to Claim 1,
wherein the distribution diagram of the correspondence table (5) is displayed on a screen.

9. An automatic analyzer (10) comprising the data processing device (1) of claim 1 as part of the automatic analyzer.

10. A system comprising the data processing device (1) of claim 1 and an automatic analyzer (10).

## Patentansprüche

1. Datenverarbeitungsvorrichtung (1), die konfiguriert ist, die folgenden Prozesse auszuführen:
Bezugnehmen (S1) auf Daten bezüglich der Messung einer Probe durch einen automatischen Analysator (10);
Bestimmen (S6) eines abnormalen Bereichsgebiets; und
Ausgeben (S8) von Informationen bezüglich eines Abnormalitätsursachenkandidaten an einen Benutzer,
**dadurch gekennzeichnet, dass** die Datenverarbeitungsvorrichtung (1) konfiguriert ist, die folgenden weiteren Prozesse auszuführen:
Berechnen (S2) einer Kombination von Werten einer Vielzahl von Faktoren basierend auf Reaktionsprozessdaten unter den Daten bezüglich der Messung der Probe, wobei die Faktoren vorbestimmte Variablen oder Messwerte sind, die Charakteristiken einer Reaktion repräsentieren;
Bezugnehmen (S5) auf eine Korrespondenztabelle (5), die ein Verteilungsdiagramm und Informationen bezüglich Abnormalitätsursachenkandidaten enthält, wobei das Verteilungsdiagramm ein normales Bereichsgebiet und eine Vielzahl von abnormalen Bereichsgebieten umfasst, die durch eine Kombination von normalen/abnormalen Bereichen der Vielzahl von Faktoren erzeugt werden, und die Abnormalitätsursachenkandidaten jedem der abnormalen Bereichsgebiete zugeordnet sind, wobei das Verteilungsdiagramm das normale Bereichsgebiet (501, 601, 701, 901), ein erstes abnormales Bereichsgebiet (502, 602, 702, 902), ein zweites abnormales Bereichsgebiet (502, 602, 702, 902) und ein drittes abnormales Bereichsgebiet (502, 602, 702, 902) umfasst, die durch mindestens eine Kombination eines normalen Bereichs (511, 611, 711) und eines abnormalen Bereichs (512, 513, 612, 613, 712) eines ersten Faktors und eines normalen Bereichs (521, 621, 721) und eines abnormalen Bereichs (522, 523, 622, 722) eines zweiten Faktors erzeugt werden, wobei das normale Bereichsgebiet (501, 601, 701, 901) durch den normalen Bereich (511, 611, 711) des ersten Faktors und den normalen Bereich (521, 621, 721) des zweiten Faktors konfiguriert ist, das erste abnormale Bereichsgebiet (502, 602, 702, 902) durch den normalen Bereich (511, 611, 711) des ersten Faktors und den abnormalen Bereich (522, 523, 622, 722) des zweiten Faktors konfiguriert ist, das zweite abnormale Bereichsgebiet (502, 602, 702, 902) durch den abnormalen Bereich (512, 513, 612, 613, 712) des ersten Faktors und den normalen Bereich (521, 621, 721) des zweiten Faktors konfiguriert ist und das dritte abnormale Bereichsgebiet (502, 602, 702, 902) durch den abnormalen Bereich (512, 513, 612, 613, 712) des ersten Faktors und den abnormalen Bereich (522, 523, 622, 722) des zweiten Faktors konfiguriert ist,
wobei das Bestimmen (S6) eines abnormalen Bereichsgebiets das Bestimmen (S6) desjenigen abnormalen Bereichsgebiets (502, 602, 702, 902) in der Korrespondenztabelle (5) ist, dem die Kombination der Werte der Vielzahl von Faktoren entspricht; und
das Ausgeben (S8) von Informationen bezüglich eines Abnormalitätsursachenkandidaten das Ausgeben (S8) der Informationen bezüglich des Abnormalitätsursachenkandidaten ist, der dem entsprechenden abnormalen Bereichsgebiet (502, 602, 702, 902) zugeordnet ist.

2. Datenverarbeitungsvorrichtung nach Anspruch 1, wobei die Korrespondenztabelle (5) so eingerichtet ist, dass jedes der abnormalen Bereichsgebiete oder jeder der Abnormalitätsursachenkandidaten mit Informationen bezüglich einer Gegenmaßnahme verbunden ist.

3. Datenverarbeitungsvorrichtung nach Anspruch 1, wobei die Korrespondenztabelle (5) im Voraus als Standardeinstellungsinformation durch einen Geschäftsbetreiber des automatischen Analysators eingestellt ist.

4. Datenverarbeitungsvorrichtung nach Anspruch 1, wobei die Korrespondenztabelle (5) aktualisiert wird, indem Informationen bezüglich der Korrespondenztabelle auf einem Bildschirm angezeigt werden und die Informationen bezüglich des Abnormalitätsursachenkandidaten basierend auf einer Operation des Benutzers auf dem Bildschirm hinzugefügt oder modifiziert werden.

5. Datenverarbeitungsvorrichtung nach Anspruch 1, wobei die Reaktionsprozessdaten Daten sind, die eine zeitliche Änderung der Absorption darstellen, und als die Variable, die einer der Faktoren ist, eine Variable verwendet wird, die einen Näherungsausdruck der Reaktionsprozessdaten konfiguriert.

6. Datenverarbeitungsvorrichtung nach Anspruch 1, wobei die Reaktionsprozessdaten Daten sind, die eine zeitliche Änderung der Absorption darstellen, und als die Variable, die einer der Faktoren ist, ein mittlerer quadratischer Fehler bezüglich einer Differenz zwischen einem Näherungsausdruck und einer Absorption in den Reaktionsprozessdaten verwendet wird.

7. Datenverarbeitungsvorrichtung nach Anspruch 1, wobei die Reaktionsprozessdaten Daten sind, die eine zeitliche Änderung der Absorption darstellen, und als der Messwert, der einer der Faktoren ist, eine Konzentration oder ein Aktivitätswert einer Komponente der Probe verwendet wird.

8. Datenverarbeitungsvorrichtung nach Anspruch 1, wobei das Verteilungsdiagramm der Korrespondenztabelle (5) auf einem Bildschirm angezeigt wird.

9. Automatischer Analysator (10), der die Datenverarbeitungsvorrichtung (1) nach Anspruch 1 als Teil des automatischen Analysators umfasst.

10. System, das die Datenverarbeitungsvorrichtung (1) nach Anspruch 1 und einen automatischen Analysator (10) umfasst.

## Revendications

1. Dispositif (1) de traitement de données configuré pour exécuter les processus suivants de :
référence (S1) à des données concernant une mesure d'un échantillon par un analyseur automatique (10) ;
détermination (S6) d'une région de plages anormales ; et
délivrance en sortie (S8) d'informations concernant une cause candidate d'anomalie à un utilisateur,
**caractérisé en ce que** le dispositif (1) de traitement de données est configuré pour exécuter les autres processus suivants :
calcul (S2) d'une combinaison de valeurs d'une pluralité de facteurs sur la base de données de processus de réaction parmi les données concernant une mesure de l'échantillon, les facteurs étant des variables prédéterminées ou des valeurs mesurées représentant des caractéristiques d'une réaction ;
référence (S5) à une table (5) de correspondances incluant un diagramme de distribution et des informations concernant des causes candidates d'anomalies, le diagramme de distribution incluant une région de plages normales et une pluralité de régions de plages anormales qui sont générées par une combinaison de plages normales/anormales de la pluralité de facteurs, et les causes candidates d'anomalies étant associées avec chacune des régions de plages anormales, dans lequel le diagramme de distribution inclut la région de plages normales (501, 601, 701, 901), une première région de plages anormales (502, 602, 702, 902), une deuxième région de plages anormales (502, 602, 702, 902), et une troisième région de plages anormales (502, 602, 702, 902) qui sont générées par au moins une combinaison d'une plage normale (511, 611, 711) et d'une plage anormale (512, 513, 612, 613, 712) d'un premier facteur et d'une plage normale (521, 621, 721) et d'une plage anormale (522, 523, 622, 722) d'un deuxième facteur, la région de plages normales (501, 601, 701, 901) étant configurée par la plage normale (511, 611, 711) du premier facteur et la plage normale (521, 621, 721) du deuxième facteur, la première région de plages anormales (502, 602, 702, 902) étant configurée par la plage normale (511, 611, 711) du premier facteur et la plage anormale (522, 523, 622, 722) du deuxième facteur, la deuxième région de plages anormales (502, 602, 702, 902) étant configurée par la plage anormale (512, 513, 612, 613, 712) du premier facteur et la plage normale (521, 621, 721) du deuxième facteur, et la troisième région de plages anormales (502, 602, 702, 902) étant configurée par la plage anormale (512, 513, 612, 613, 712) du premier facteur et la plage anormale (522, 523, 622, 722) du deuxième facteur, dans lequel
la détermination (S6) d'une région de plages anormales détermine (S6) la région de plages anormales (502, 602, 702, 902) dans la table (5) de correspondances à laquelle la combinaison des valeurs de la pluralité de facteurs correspond ; et
la délivrance en sortie (S8) d'informations concernant une cause candidate d'anomalie délivre en sortie (S8) les informations concernant la cause candidate d'anomalie associée avec la région de plages anormales (502, 602, 702, 902) correspondante.

2. Dispositif de traitement de données selon la revendication 1,
dans lequel la table (5) de correspondances est définie de façon à ce que chacune des régions de plages anormales ou chacune des causes candidates d'anomalies soit associée avec des informations concernant une contre-mesure.

3. Dispositif de traitement de données selon la revendication 1,
dans lequel la table (5) de correspondances est définie au préalable comme des informations de paramétrage par défaut par un opérateur d'exploitation de l'analyseur automatique.

4. Dispositif de traitement de données selon la revendication 1,
dans lequel la table (5) de correspondances est mise à jour en affichant des informations concernant la table de correspondances sur un écran et en ajoutant ou en modifiant les informations concernant la cause candidate d'anomalie sur la base d'une opération de l'utilisateur sur l'écran.

5. Dispositif de traitement de données selon la revendication 1,
dans lequel les données de processus de réaction sont des données représentant un changement temporel d'absorbance, et
comme la variable qui est un des facteurs, une variable configurant une expression approchée des données de processus de réaction est utilisée.

6. Dispositif de traitement de données selon la revendication 1,
dans lequel les données de processus de réaction sont des données représentant un changement temporel d'absorbance, et
comme la variable qui est un des facteurs, une erreur quadratique moyenne concernant une différence entre une expression approchée et une absorbance dans les données de processus de réaction est utilisée.

7. Dispositif de traitement de données selon la revendication 1,
dans lequel les données de processus de réaction sont des données représentant un changement temporel d'absorbance, et
comme la valeur mesurée qui est un des facteurs, une concentration ou une valeur d'activité d'un composant de l'échantillon est utilisée.

8. Dispositif de traitement de données selon la revendication 1,
dans lequel le diagramme de distribution de la table (5) de correspondances est affiché sur un écran.

9. Analyseur automatique (10) comprenant le dispositif (1) de traitement de données selon la revendication 1 comme partie de l'analyseur automatique.

10. Système comprenant le dispositif (1) de traitement de données selon la revendication 1 et un analyseur automatique (10).
